# EUROPEAN PATENT SPECIFICATION

(11) **EP 2 340 022 B1**
(45) Date of publication and mention of the grant of the patent: **20.11.2013**
(21) Application number: 09787058.8
(22) Date of filing: 31.08.2009
(51) Int. Cl.: A61K 31/4409, A61K 31/4709, A61K 31/496, A61K 31/4965, A61K 31/5377, A61K 31/541, A61K 45/06, A61P 31/06

(54) **COMBINATION THERAPY FOR TUBERCULOSIS**
KOMBINATIONSTHERAPIE GEGEN TUBERKULOSE
THÉRAPIE COMBINÉE POUR LA TUBERCULOSE

(30) Priority: 03.09.2008 US 93879 P
(43) Date of publication of application: 06.07.2011
(73) Proprietor: Pfizer Inc., New York, NY 10017 (US); The Johns Hopkins University, Baltimore, MD 21218 (US)
(72) Inventor: BRICKNER, Steven Joseph, Ledyard Connecticut 06339 (US); NUERMBERGER, Eric, Baltimore Maryland 21231 (US); STOVER, Charles Kendall, Gaithersburg Maryland 20878 (US)
(74) Representative: Johansson, Lars-Erik
(86) International application number: PCT/IB2009/053796
(87) International publication number: WO 2010/026526

(56) References cited:
- US-A- 5 880 118
- CYNAMON M H ET AL: "Activities of several novel oxazolidinones against Mycobacterium tuberculosis in a murine model" ANTIMICROBIAL AGENTS AND CHEMOTHERAPY, vol. 43, no. 5, May 1999 (1999-05), pages 1189-1191, XP002557656 ISSN: 0066-4804
- BALLELL L ET AL: "NEW SMALL-MOLECULE SYNTHETIC ANTIMYCOBACTERIALS" ANTIMICROBIAL AGENTS AND CHEMOTHERAPY, AMERICAN SOCIETY FOR MICROBIOLOGY, WASHINGTON, DC, US, vol. 49, no. 6, 1 June 2005 (2005-06-01), pages 2153-2163, XP008056050 ISSN: 0066-4804

## Description

### Field of the Invention

The present invention relates to methods of treating tuberculosis, including multi-drug resistant varieties and latent tuberculosis. More particularly, the present invention relates to use of a compound of formula (I), (S)-N-[[3-[3-fluoro-4-(4-thiomorpholinyl)phenyl]-2-oxo-5-oxazolidinyl]methyl]acetamide, or a pharmaceutically acceptable salt thereof in combination with at least two anti-tuberculin agents in the manufacture of a medicament for the treatment of tuberculosis. The present invention also relates to a pharmaceutical composition comprising: I) a therapeutically effective amount of a compound of formula (I) or a pharmaceutically acceptable salt thereof, (ii) a therapeutically effective amount of at least two agents useful in the treatment of tuberculosis and (iii) one or more pharmaceutically acceptable carriers or vehicles.

### Background of the Invention

Tuberculosis (TB) kills approximately 1.6 million people worldwide each year, making it the second leading killer of adults behind HIV. Nearly 500,000 new cases of multidrug-resistant (MDR) TB occur each year, and the recent emergence of extensively drug-resistant (XDR-TB) TB portends new epidemics of untreatable TB. (See Dorman, S. E. et al., Nat.Med 13:295-298 2007, Zignol, M. et al., J Infect.Dis 194:479-485, 2006). New drugs with potent anti-tuberculosis activity, especially against non-multiplying persisters, are needed to shorten the duration of treatment for TB and thereby facilitate the global implementation of directly-observed therapy. (See O'Brien, R. J. et al., Am.J.Respir. Crit Care Med. 163:1055-1058, 2001).

Currently, the treatment of drug-sensitive tuberculosis consists of administering a combination of at least the following drugs, isoniazid, rifampin, and pyrazinamide. For effective treatment, the above-mentioned drugs are given to a patient in an initial phase of treatment for 8 weeks, during which the drugs are used in combination to kill the rapidly multiplying population of *Mycobacterium tuberculosis* as well as to prevent the emergence of drug resistance. The initial phase of treatment is followed by a continuation or a sterilization phase for 18 weeks during which two or more sterilizing drugs (e.g. isoniazid and rifampin) are given to kill the intermittently dividing population (non-multiplying persisters) of *Mycobacterium tuberculosis* (see, for example, Ballell h et al, Antimicrob Agents Chemother 49: 2153-2163, 2005).

While the above-mentioned combination of drugs together provide treatment against sensitive *Mycobacterium tuberculosis* infection in 4 to 6 months time, such a combination therapy is not always successful, especially in patients harboring drug resistant strains. Also, the long duration of treatment consisting of six months may lead to unpleasant side effects. Further, compliance with the relatively long course of treatment is generally poor. Such non-compliance may lead to treatment failure resulting in development of drug resistance.

The oxazolidinones comprise a class of protein synthesis inhibitors that block translation by preventing formation of the initiation complex (for mechanism, see K. Leach et al., Molecular Cell, 26:4, 460-462, 2007). Linezolid (LZD, Zyvox^{®}), the only marketed oxazolidinone, has activity against Gram-positive bacteria and is currently approved for use in complicated skin and skin structure infections and hospital-acquired pneumonia (Zyvox^{®} package insert). However, it is also active against many mycobacterial species, including *Mycobacterium tuberculosis,* for which its MIC ranges from 0.125-1 µg/mL, with an MIC₅₀ of 0.5 µg/mL and an MIC₉₀ of 1 µg/mL. (See Alcala, L., et al., Antimicrob Agents Chemother 47:416-417, 2003; Cynamon, M. H., et al., Antimicrob Agents Chemother 43:1189-1191, 1999; Fattorini, L., et al., Antimicrob Agents Chemother 47:360-362, 2003). As a result, LZD has been used outside of labeled indications to treat recalcitrant cases of MDR- and XDR-TB. Although several case series suggest that LZD may contribute to successful sputum culture conversion in such cases, its individual activity in TB patients and its precise contribution to combination regimens remain unclear. These studies also demonstrate that the duration of LZD administration may be limited by hematologic and neurologic toxicity that can occur with long-term administration. (See Fortun, J., et al., 56:180-185, 2005; Park, I. N., et al., Antimicrob Chemother 58:701-704, 2006; Zignol, M., et al., J Infect.Dis 194:479-485, 2006). Therefore, new oxazolidinones with more potent in vivo activity against *Mycobacterium tuberculosis* and lower risk of toxicity with prolonged administration are desirable.

Oxazolidinones with more potent activity against *Mycobacterium tuberculosis* have previously been described. (See Barbachyn, M. R. et al., J Med Chem. 39:680-685, 1996; Sood, R., et al., Antimicrob Agents Chemother 49:4351-4353, 2005). The antituberculosis activity of the compound of formula (I), (S)-N-[[3-[3-fluoro-4-(4-thiomorpholinyl)phenyl]-2-oxo-5-oxazolidinyl]methyl]acetamide, was first described in 1996. (See Barbachyn, M. R., et al., J Med Chem. 39:680-685,1996). Subsequent experiments in a murine model found the compound of formula (I) to be more active than LZD when both drugs were administered at 100 mg/kg, but the clinical relevance of this LZD dose was not established and the activities of the compound of formula (I), and LZD were not clearly different when compared at lower doses. (See Cynamon, M. H., et al., Antimicrob Agents Chemother 43:1189-1191, 1999). Moreover, although the compound of formula (I) appeared to have modest activity when combined with rifampin (RIF) in an acute (early) infection model, the compound of formula (I) had no additional activity when combined with isoniazid (INH). (Cynamon, M. H., et al., Antimicrob Agents Chemother 43:1189-1191, 1999.) Most importantly, the activity of the compound of formula (I), whether alone or in combination with RIF or INH, was only evaluated over the initial 4 weeks of treatment which is insufficient to assess the activity of a compound or combination of agents against non-multiplying persisters which, in turn, ultimately determines the duration of treatment necessary for cure (i.e., prevention of relapse after completion of treatment).

Hence, there is an urgent need to develop newer regimens that can be used to prevent, treat and/or reduce tuberculosis and/or eliminate the threat of multi-drug resistant tuberculosis or shorten the duration of treatment.

### Summary of the Invention

The present invention relates to use of a compound of formula (I) or a pharmaceutically acceptable salt thereof:

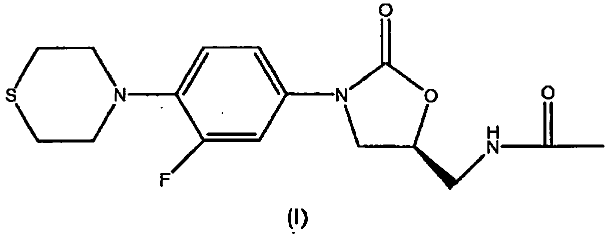

in combination with at least two anti-tuberculin agents in the manufacture of a medicament for treatment of tuberculosis. In one embodiment, the at least two agents are selected from the group consisting of isoniazid, rifampin, rifapentine, rifabutin, pyrazinamide, ethambutol, streptomycin, kanamycin, amikacin, moxifloxacin, gatifloxacin, levofloxacin, ofloxacin, ciprofloxacin, capreomycin, ethionamide, cycloserine, para-aminosalicylic acid, thiacetazone, clarithromycin, amoxicillin-clavulanic acid, imipenem, meropenem, viomycin, terizidone, clofazimine, TMC207, PA-824, OPC-7683, LL-3858 and SQ-109.

In another embodiment, one of said at least two agents is selected from the group consisting of isoniazid, rifampin, rifapentine, rifabutin, pyrazinamide, moxifloxacin, gatifloxacin, levofloxacin, ofloxacin, ciprofloxacin, clofazimine and ethambutol.

In yet another embodiment, one of said at least two agents is pyrazinamide.

In yet another embodiment, one of said at least two agents is rifampin.

In yet another embodiment, one of said at least two agents is rifapentine.

In yet another embodiment, one of said at least two agents is PA-824.

In yet another embodiment, one of said at least two agents is OPC-67683

In yet another embodiment, one of said at least two agents is TMC-207.

In yet another embodiment, one of said at least two agents is selected from the group consisting of moxifloxacin, gatifloxacin, levofloxacin, and ofloxacin.

In yet another embodiment one of said at least two agents is moxifloxacin.

In a specific embodiment, said at least two agents are pyrazinamide and rifampin.

In yet another specific embodiment, said at least two agents are pyrazinamide, rifampin and isoniazid.

In yet another specific embodiment, said at least two agents are pyrazinamide and rifapentine.

In yet another specific embodiment, said at least two agents are pyrazinamide, rifapentine, and isoniazid.

In a specific embodiment, said at least two agents are pyrazinamide and moxifloxacin.

In yet another specific embodiment, said at least two agents are pyrazinamide, moxifloxacin, and rifampin.

In yet another specific embodiment, said at least two agents are pyrazinamide, moxifloxacin, and rifapentine.

In yet another specific embodiment, said at least two agents are PA-824 and pyrazinamide.

In yet another specific embodiment, said at least two agents are PA-824, pyrazinamide and an agent selected from the group consisting of streptomycin, kanamycin, amikacin and capreomycin.

In yet another specific embodiment, said at least two agents are PA-824 and moxifloxacin.

In yet another specific embodiment, said at least two agents are PA-824, moxifloxacin and an agent selected from the group consisting of streptomycin, kanamycin, amikacin and capreomycin.

In yet another specific embodiment, said at least two agents are OPC-67683 and pyrazinamide.

In yet another specific embodiment, said at least two agents are OPC-67683, pyrazinamide and an agent selected from the group consisting of streptomycin, kanamycin, amikacin and capreomycin.

In yet another specific embodiment, said at least two agents are OPC-67683 and moxifloxacin.

In yet another specific embodiment, said at least two agents are OPC-67683, moxifloxacin and an agent selected from the group consisting of streptomycin, kanamycin, amikacin and capreomycin.

In yet another specific embodiment, said at least two agents are TMC-207 and pyrazinamide.

In yet another specific embodiment, said at least two agents are TMC-207, pyrazinamide and moxifloxacin or isoniazid.

In yet another embodiment, said tuberculosis comprises active tuberculosis or latent tuberculosis.

In yet another embodiment, said active tuberculosis comprises drug sensitive, mono-drug resistant, multi-drug-resistant tuberculosis (MDR) or extensively drug-resistant tuberculosis (XDR).

In yet another embodiment, the use of the present invention completely eradicates drug-sensitive tuberculosis, mono-drug resistant, multi-drug-resistant tuberculosis, and extensively drug-resistant tuberculosis (XDR) on completion of the treatment.

In yet another embodiment, said tuberculosis is caused by a *Mycobacterium* infection selected from the group consisting of *Mycobacterium tuberculosis, Mycobacterium bovis* or other related mycobacterial specles.

In yet another embodiment, the use of the present invention prevents relapse of the *Mycobacterium* infection after completion of the treatment.

In yet another embodiment, the compound of formula (I) and pharmaceutically acceptable salts thereof is administered orally.

In yet another embodiment, said at least two agents are each administered orally.

In yet another embodiment, said at least two agents are administered together in a composition.

In yet another embodiment, said at least two agents are administered separately.

In yet another embodiment, said at least two agents are administered together and another agent useful for the treatment of tuberculosis is administered separately.

In yet another embodiment, the compound of formula (I) and pharmaceutically acceptable salts thereof is administered once per day (QD) or twice per day (BID).

In yet another embodiment, the compound of formula (I) and pharmaceutically acceptable salts thereof is administered once per week, twice per week, thrice per week or every other day.

In one embodiment, the compound of formula (I) or a pharmaceutically acceptable salt thereof is administered between about 10 mg to about 2000 mg.

In yet another embodiment, the compound of formula (I), or a pharmaceutically acceptable salt thereof, is administered between about 250 mg to about 1000 mg.

In yet another embodiment, the compound of formula (I), or a pharmaceutically acceptable salt thereof, is administered between about 600 mg to about 1000 mg.

In yet another embodiment, the compound of formula (I) or a pharmaceutically acceptable salt thereof is administered between about 25 mg to about 1000 mg.

In yet another embodiment, the compound of formula (I) or a pharmaceutically acceptable salt thereof is administered between about 50 mg to about 500 mg.

In yet another embodiment, the compound of formula (I) or a pharmaceutically acceptable salt thereof Is administered between about 100 mg to about 500 mg.

The present invention also relates to use of a compound of formula (I) or a pharmaceutically acceptable salt thereof: 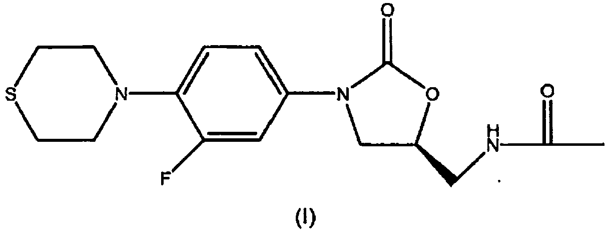 in combination with at least one anti-tuberculin agent in the manufacture of a medicament for treating tuberculosis after a subject has undergone an initial phase of treatment, for tuberculosis.

In one embodiment, said at least one agent is selected from the group consisting of rifampin, rifapentine, rifabutin, pyrazinamide, ethambutol, streptomycin, kanamycin, amikacin, moxifloxacin, gatifloxacin, levofloxacin, ofloxacin, ciprofloxacin, capreomycin, ethionamide, cycloserine, para-aminosalicylic acid, thiacetazone, clarithromycin, amoxicillin-clavulanic acid, imipenem, meropenem, clofazimine, viomycin, terizidone, TMC207, PA-824, OPC-7683, LL-3858 and SQ-109.

In yet another embodiment, said at least one agent is selected from the group consisting of rifampin, rifapentine, rifabutin, pyrazinamide, moxifloxacin, gatifloxacin, levofloxacin, ofloxacin, and ethambutol.

In a specific embodiment, said at least one agent is pyrazinamide.

In a specific embodiment, said at least one agent is rifampin.

In a specific embodiment, said at least one agent Is rifapentine.

In a specific embodiment, said at least one agent is PA-824.

In a specific embodiment, said at least one agent is OPC-7683.

In a specific embodiment, said at least one agent is TMC-207.

In a specific embodiment, said at least one agent is selected from the group consisting of moxifloxacin, gatifloxacin, levofloxacin, and ofloxacin.

In a specific embodiment, said at least one agent is moxifloxacin.

In yet another embodiment, said tuberculosis comprises active tuberculosis or latent tuberculosis.

In yet another embodiment, said active tuberculosis comprises drug-sensitive tuberculosis, mono-drug resistant, multi-drug-resistant tuberculosis (MDR) or extensively drug-resistant tuberculosis (XDR).

In yet another embodiment, the use of the present invention completely eradicates drug-sensitive tuberculosis, mono-drug resistant, multi-drug-resistant tuberculosis, and extensively drug-resistant tuberculosis (XDR) on completion of the treatment.

In yet another embodiment, said tuberculosis is caused by a *Mycobacterium* infection selected from the group consisting of *Mycobacterium tuberculosis, Mycobacterium bovis* or other related mycobacterial species.

In yet another embodiment, the use of the present invention prevents relapse of the *Mycobacterium* infection after completion of the treatment.

In yet another embodiment, the compound of formula (I) and pharmaceutically acceptable salts thereof is administered orally.

In yet another embodiment, said at least one agent is administered orally.

In yet another embodiment, the compound of formula (I) and pharmaceutically acceptable salts thereof and said at least one agent are administered together in a composition.

In yet another embodiment, the compound of formula (I) and pharmaceutically acceptable salts thereof and said at least one agent are administered separately.

In yet another embodiment, the compound of formula (I) and pharmaceutically acceptable salts thereof is administered once per day (QD) or twice per day (BID).

In yet another embodiment, the compound of formula (I) and pharmaceutically acceptable salts thereof is administered once per week, twice per week, thrice per week or every other day.

In one embodiment, the compound of formula (I) or a pharmaceutically acceptable salt thereof is administered between about 10 mg to about 2000 mg.

In yet another embodiment, the compound of formula (I) or a pharmaceutically acceptable salt thereof is administered between about 25 mg to about 1000 mg.

In yet another embodiment, the compound of formula (I) or a pharmaceutically acceptable salt thereof is administered between about 50 mg to about 500 mg.

In yet another embodiment, the compound of formula (I) or a pharmaceutically acceptable salt thereof is administered between about 100 mg to about 500 mg.

The present invention also relates to a pharmaceutical composition comprising:
i) a therapeutically effective amount of a compound of formula (I) or a pharmaceutically acceptable salt thereof: 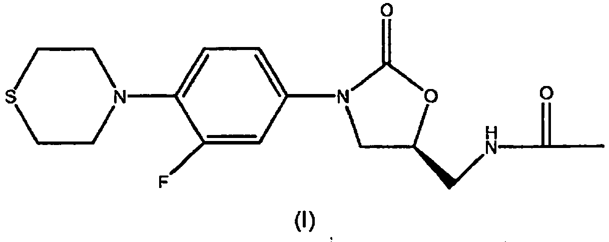
ii) a therapeutically effective amount of at least two agents useful in the treatment of tuberculosis and
iii) one or more pharmaceutically acceptable carriers or vehicles.

In yet another embodiment, said at least two agents are selected from the group consisting of isoniazid, rifampin, rifapentine, rifabutin, pyrazinamide, ethambutol, streptomycin, kanamycin, amikacin, moxifloxacin, gatifloxacin, levofloxacin, ofloxacin, ciprofloxacin, capreomycin, ethionamide, cycloserine, para-aminosalicylic acid, thiacetazone, clarithromycin, amoxicillin-clavulanic acid, imipenem, meropenem, viomycin, terizidone, clofazimine, TMC207, PA-824, OPC-7683, LL-3858 and SQ-109.

In yet another embodiment, said at least two agents are selected from the group consisting of isoniazid, rifampin, rifapentine, rifabutin, pyrazinamide, moxifloxacin, gatifloxacin, levofloxacin, ofloxacin, and ethambutol.

In yet another embodiment, the pharmaceutical composition comprises about 10 mg to about 2000 mg of compound of formula (I) or a pharmaceutically acceptable salt thereof.

In yet another embodiment, the pharmaceutical composition comprises about 250 mg to about 1000 mg of compound of formula (I) or a pharmaceutically acceptable salt thereof.

In yet another embodiment, the pharmaceutical composition comprises about 600 mg to about 1000 mg of compound of formula (I) or a pharmaceutically acceptable salt thereof.

In yet another embodiment, the pharmaceutical composition comprises about 25 mg to about 1000 mg of compound of formula (I) or a pharmaceutically acceptable salt thereof.

In yet another embodiment, the pharmaceutical composition comprises about 50 mg to about 500 mg of compound of formula (I) or a pharmaceutically acceptable salt thereof.

Additionally, any formulation, including the combinations below, may contain from 250 mg to 1000 mg of the compound of formula (I) or a pharmaceutically acceptable salt thereof or from 600 mg to 1000 mg of compound of formula (I) or a pharmaceutically acceptable salt thereof.

In yet another embodiment, the pharmaceutical composition comprises about 100 mg to about 500mg of compound of formula (I) or a pharmaceutically acceptable salt thereof.

In yet another embodiment, the pharmaceutical composition comprises about 100 mg to about 500mg of compound of formula (I) or a pharmaceutically acceptable salt thereof and about 600 mg rifampin.

In yet another embodiment, the pharmaceutical composition comprises about 100 mg to about 500 mg of compound of formula (I) or a pharmaceutically acceptable salt thereof and about 300 mg of isoniazid.

In yet another embodiment, the pharmaceutical composition comprises about 100 mg to about 500mg of compound of formula (I) or a pharmaceutically acceptable salt thereof and about 300 mg of isoniazid and about 600 mg of rifampin.

In yet another embodiment, the pharmaceutical composition comprises about 100 mg to about 500 mg of compound of formula (I) or a pharmaceutically acceptable salt thereof, about 300 mg of isoniazid, about 600 mg of rifampin and about 20-25 mg/kg to about 50-70 mg/kg of pyrazinamide.

In yet another embodiment, the pharmaceutical composition comprises about 100 mg to about 500 mg of a compound of formula (I) or a pharmaceutically acceptable salt thereof and about 10-15 mg/kg to about 20-30 mg/kg of isoniazid.

In yet another embodiment, the pharmaceutical composition comprises about 100 mg to about 500 mg of a compound of formula (I) or a pharmaceutically acceptable salt thereof and about 10-15 mg/kg to about 20-30 mg/kg of isoniazid and about 10 mg/kg to about 20 mg/kg of rifampin.

In yet another embodiment, the pharmaceutical composition comprises about 100 mg to about 500 mg of a compound of formula (I) or a pharmaceutically acceptable salt thereof, about 10-15 mg/kg to about 20-30 mg/kg of isoniazid, about 10 mg/kg to about 20 mg/kg of rifampin and about 15-30 mg/kg to about 50 mg/kg pyrazinamide.

In yet another embodiment, the pharmaceutical composition comprises about 50 mg to about 250 mg of a compound of formula (I) or a pharmaceutically acceptable salt thereof, about 75 mg isoniazid, about 150 mg of rifampin and about 400 mg of pyrazinamide.

In yet another embodiment, the pharmaceutical composition comprises about 25 mg to about 250 mg of a compound of formula (I) or a pharmaceutically acceptable salt thereof, about 300 mg of rifampin.

There is also disclosed an article of manufacture comprising:
a packaged composition comprising:
   (a)
      i) a therapeutically effective amount of a compound of formula (I) or a pharmaceutically acceptable salt thereof: 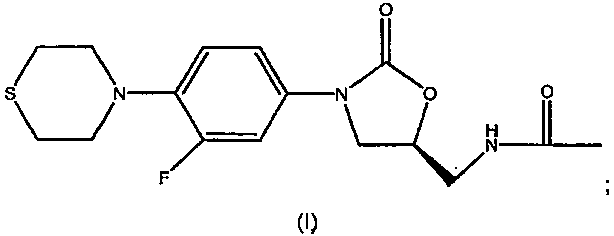
      ii) a therapeutically effective amount of at least one agent useful in the treatment of tuberculosis and
      iii) one or more pharmaceutically acceptable carriers or vehicles
   (b) an insert providing instructions for administration of the packaged composition of (a) to treat tuberculosis; and
   (c) a container for (a) and (b).

There is also disclosed a pharmaceutical package for treating tuberculosis in a mammal which comprises a compound of formula (I) or a pharmaceutically acceptable salt thereof: 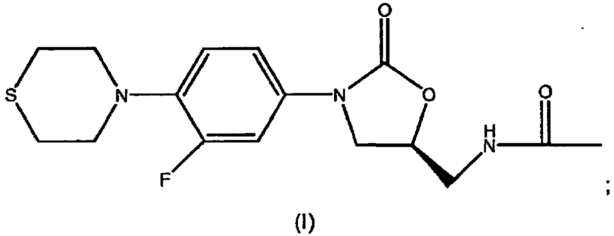 and an insert providing instructions for administering said composition in combination with at least one agent useful in the treatment of tuberculosis.

These and other aspects, advantages, and features of the invention will become apparent for the following detailed description of the invention.

### Detailed Description of the invention

The present invention relates to use of a compound of formula (I) or a pharmaceutically acceptable salt thereof: 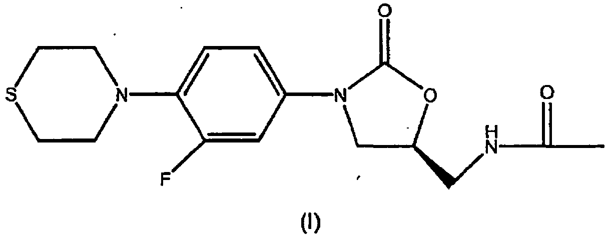 in combination with at least two anti-tuberculin agents in the manufacture of a medicament for the treatment of tuberculosis.

The compound of formula (I) of the invention is disclosed in U.S. Patent No. 5,880,118, (incorporated in its entirety herein by reference) in Example 1, (S)-N-[[3-[3-fluoro-4-(4-thiomorpholinyl)phenyl]-2-oxo-5-oxazoildinyl]methyl]acetamide. As described in more detail below, the compound of formula (I) of the invention may be administered as the free base or in the form of a salt thereof.

The phrase "pharmaceutically acceptable salt(s)", as used herein, unless otherwise indicated, includes salts of acidic or basic groups which may be present in the compounds of formula (I).

For example, the compounds of formula (I) that are basic in nature are capable of forming a wide variety of salts with various inorganic and organic acids. The acids that may be used to prepare pharmaceutically acceptable acid addition salts of such basic compounds of those that form non-toxic acid addition salts, i.e., salts containing pharmacologically acceptable anions, such as the hydrochloride, hydrobromide, hydroiodide, nitrate, sulfate, bisulfate, phosphate, acid phosphate, isonicotinate, lactate, salicylate, citrate, acid citrate, tartrate, pantothenate, bitartrate, ascorbate, succinate, maleate, fumarate, gluconate, glucuronate, saccharate, formate, benzoate, glutamate, methanesulfonate, ethanesulfonate, benzenesulfonate, p-toluenesulfonate and pamoate [i.e., 1,1'-methylene-bis-(2-hydroxy-3-naphthoate)] salts.

Examples of salts include, but are not limited to, acetate, acrylate, benzenesulfonate, benzoate (such as chlorobenzoate, methylbenzoate, dinitrobenzoate, hydroxybenzoate, and methoxybenzoate), bicarbonate, bisulfate, bisulfite, bitartrate, borate, bromide, butyne-1,4-dioate, calcium edetate, camsylate, chloride, caproate, caprylate, citrate, decanoate, dihydrogenphosphate, edetate, edislyate, estolate, esylate, ethylsuccinate, formate, fumarate, gluceptate, gluconate, glutamate, glycollate, glycollylarsanilate, heptanoate, hexyne-1,6-dioate, hexylresorcinate, hydrabamine, hydrobromide, hydrochloride, γ-hydroxybutyrate, iodide, isobutyrate, isothionate, lactate, lactobionate, laurate, malate, maleate, malonate, mandelate, mesylate, metaphosphate, methane-sulfonate, methylsulfate, monohydrogenphosphate, mucate, napsylate, naphthalene-1-sulfonate, naphthalene-2-sulfonate, nitrate, oleate, oxalate, pamoate (embonate), palmitate, pantothenate, phenylacetates, phenylbutyrate, phenylpropionate, phthalate, phospate/diphosphate, polygalacturonate, propanesulfonate, propionate, propiolate, pyrophosphate, pyrosulfate, salicylate, stearate, subacetate, suberate, succinate, sulfate, sulfonate, sulfite, tannate, tartrate, teoclate, tosylate, triethiodode, and valerate salts.

The invention also relates to base addition salts of the compounds of formula (I). The chemical bases that may be used as reagents to prepare pharmaceutically acceptable base salts of the compounds of formula (I) that are acidic in nature are those that form non-toxic base salts with such compounds. Such non-toxic base salts include, but are not limited to, those derived from such pharmacologically acceptable cations such as alkali metal cations (e.g., potassium and sodium) and alkaline earth metal cations (e.g., calcium and magnesium), ammonium or water-soluble amine addition salts such as N-methylglucamine-(meglumine), and the lower alkanolammonium and other base salts of pharmaceutically acceptable organic amines.

Hemisalts of acids and bases may also be formed, for example, hemisulphate and hemicalcium salts.

For a review on suitable salts, see Handbook of Pharmaceutical Salts: Properties, Selection, and Use by Stahl and Wermuth (Wiley-VCH, 2002). Methods for making pharmaceutically acceptable salts of compounds of formula (I) of the invention are known to one of skill in the art.

As used herein the terms ""formula (I)" and "formula (I) or pharmaceutically acceptable salts thereof'" are defined to include all forms of the compound of formula (I), including isomers, crystalline and non-crystalline forms, isomorphs, polymorphs, metabolites, solvates, hydrates and prodrugs thereof.

The term "solvate" is used herein to describe a noncovalent or easily reversible combination between solvent and solute, or dispersion means and disperse phase. It will be understood that the solvate can be in the form of a solid, slurry (e.g., a suspension or dispersion), or solution. Non-limiting examples of solvents include ethanol, methanol, propanol, acetonitrile, dimethyl ether, diethyl ether, tetrahydrofuan, methylene chloride, and water. The term "hydrate" is employed when said solvent is water.

A currently accepted classification system for organic hydrates is one that defines isolated site, or channel hydrates - see Polymorphism in Pharmaceutical Solids by K. R. Morris (Ed. H. G. Brittain, Marcel Dekker, 1995). Isolated site hydrates are ones in which the water molecules are isolated from direct contact with each other by intervening organic molecules. In channel hydrates, the water molecules lie in lattice channels where they are next to other water molecules.

When the solvent or water is tightly bound, the complex will have a well-defined stoichiometry independent of humidity. When, however, the solvent or water is weakly bound, as in channel solvates and hygroscopic compounds, the water/solvent content will be dependent on humidity and drying conditions. In such cases, non-stoichiometry will be the norm.

There are also disclosed prodrugs of the compounds of formula (I). Thus certain derivatives of compounds of formula (I) which may have little or no pharmacological activity themselves can, when administered into or onto the body, be converted into compounds of formula (I) having the desired activity, for example, by hydrolytic cleavage. Such derivatives are referred to as "prodrugs". Further information on the use of prodrugs may be found In Pro-drugs as Novel Delivery Systems, Vol. 14, ACS Symposium Series (T. Higuchi and W. Stella) and Bioreversible Carriers In Drug Design, Pergamon Press, 1987 (Ed. E. B. Roche, American Pharmaceutical Association).

Prodrugs can, for example, be produced by replacing appropriate functionalities present in the compounds of formula (I) with certain moieties known to those skilled in the art as 'pro-moieties' as described, for example, in Design of Prodrugs by H. Bundgaard (Elsevier, 1985).

Some non-limiting examples of prodrugs include:
(i) where the compound of formula (I) contains a carboxylic acid functionality which is functionalized into a suitably metabolically labile group (esters, carbamates, etc.) compound of formula (I);
(ii) where the compound of formula (I) contains an alcohol functionality which is functionalized into a suitably metabolically labile group (ethers, esters, carbamates, acetals, ketals, etc.) compound of formula (I); and
(iii) where the compound of formula (I) contains a primary or secondary amino functionality, or an amide which are functionalized into a suitably metabolically labile group, e.g., a hydrolysable group (amides, carbamates, ureas, phosphonates, sulfonates, etc.) compound of formula (I).

Further examples of replacement groups in accordance with the foregoing examples and examples of other prodrug types may be found in the aforementioned references.

The compounds of the formula (I) of the invention may exhibit the phenomena of tautomerism and structural isomerism. For example, the compounds of formula (I) of the invention may exist in several tautomeric forms, including the enol and imine form, and the keto and enamine form and geometric isomers and mixtures thereof. All such tautomeric forms are included within the scope of compounds of formula (I) of the invention. Tautomers exist as mixtures of a tautomeric set in solution. In solid form, usually one tautomer predominates. Even though one tautomer may be described, the present invention includes all tautomers of the compounds of formula (I) of the invention.

The present invention also includes isotopically-labeled compounds, which are identical to those recited in formula (I) above, but for the fact that one or more atoms are replaced by an atom having an atomic mass or mass number different from the atomic mass or mass number usually found in nature. Examples of isotopes that may be incorporated into compounds of formula (I) include isotopes of hydrogen, carbon, nitrogen, oxygen, phosphorus, fluorine and chlorine, such as, but not limited to, ²H, ³H, ¹³C, ¹⁴C, ¹⁵N, ¹⁸O,¹⁷O, ³⁵S and ¹⁸F. Certain isotopically-labeled compounds of formula (I) of the invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically-labeled compounds of formula (I) of the invention may generally be prepared by carrying out the procedures disclosed in the Schemes and/or in the Examples and Preparations below, by substituting an isotopically-labeled reagent for a non-isotopically-labeled reagent.

The compounds of formula (I) of the invention may exhibit polymorphism. Polymorphs of the compounds of formula (I) of the invention may be prepared by crystallization of a compound of formula (I) of the invention under various conditions. For example, there may be employed various solvents (including water) or different solvent mixtures for recrystallization; crystallization at different temperatures; various modes of cooling ranging from very fast to very slow cooling during crystallization. Polymorphs may also be obtained by heating or melting a compound of formula (I) of the invention followed by gradual or fast cooling. The presence of polymorphs may be determined by solid probe NMR spectroscopy, IR spectroscopy, differential scanning calorimetry, powder X-ray diffraction or other such techniques.

The minimum amount of the compound of formula (I) of the invention to be administered is an effective amount. The term "effective amount" means the amount of a compound of formula (I) of the invention which prevents the onset of, alleviates the symptoms of, stops the progression of, and/or eliminates a TB infection in a mammal, e.g., a human.

A therapeutically effective amount of the compound of formula (I) of the invention was found to possess the desired antitubercular properties described below. However, a synergistic effect is observed when the compound of formula (I) of the invention is administered in combination with at least two agents useful in the treatment of tuberculosis.

By synergistic effect it is meant that the therapeutic effect of administering a compound of formula (I) of the invention and the at least two agents useful in the treatment of tuberculosis, is greater than the therapeutic effect obtained on administration of the effective amount of either compound of the formula (I) of the invention alone, or the therapeutic effective amount of the at least two agents useful in the treatment of tuberculosis administered individually or in combination.

Such synergy is advantageous in that it may allow for administration of each of the components in the combination in an amount less than that would be required if administered individually which may reduce the likelihood of adverse events or unpleasant side effects. Alternatively, such synergy may shorten the duration of treatment for TB.

Thus, administration of both the compound of formula (I) of the invention and the at least two agents useful in the treatment of tuberculosis, for example, at least two of the compounds selected from the group consisting of isoniazid, rifampin, rifapentine, rifabutin, pyrazinamide, moxifloxacin, gatifloxacin, levofloxacin, ofloxacin, ciprofloxacin, and ethambutol was found to produce an effect, which results in improved treatment of tuberculosis as compared to the effect when the compound of formula (I) of the invention alone, when the at least two agents useful in the treatment of tuberculosis administered individually or when the at least two agents useful in the treatment of tuberculosis are administered in combination with one another.

In one embodiment of the invention, administration of both the compound of formula (I) of the invention or a pharmaceutically acceptable salt thereof and at least two of the compounds selected from the group consisting of isoniazid, rifampin, rifapentine, rifabutin, pyrazinamide, moxifloxacin, gatifloxacin, levofloxacin, ofloxacin, ciprofloxacin, and ethambutol was found to produce an effect, which results in complete eradication of tuberculosis compared with incomplete eradication when the compound of formula (I) of the invention or the at least two agents useful in the treatment of tuberculosis are administered individually or in combination with one another.

The term "complete eradication" means no culturable mycobacterium could be observed in the target organ, i.e. lungs of the infected mammals, after the treatment regimen with the combination of the present invention. It is noted that at the end of treatment of infected mammals with the existing drug regimen, i.e. a combination of the at least two agents useful in the treatment of tuberculosis, isoniazid, pyrazinamide and rifampin, a significantly culturable amount of tubercule bacilli is recovered from the target organ i.e, lungs. This is evident from the data in Table 3 below. It is further noted that even after treatment for 4 months with the standard of care, 2 months of isoniazid, pyrazinamide and rifampin followed by 2 months of isoniazid and rifampin, 90% of mice relapse after completion of treatment, meaning that viable bacilli remain at the completion of treatment, even if they cannot be cultured at the time of treatment completion, which is evident from the data in Table 4.

In one embodiment of the present invention, the at least two agents useful in the treatment of tuberculosis used in conjunction with a compound of formula (I) and pharmaceutical compositions of the invention described herein are as follows: isoniazid, rifampin, rifapentine, rifabutin, pyrazinamide, ethambutol, streptomycin, kanamycin, amikacin, moxifloxacin, gatifloxacin, levofloxacin, ofloxacin, ciprofloxacin, capreomycin, ethionamide, cycloserine, para-aminosalicylic acid, thiacetazone, clarithromycin, amoxicillin-clavulanic acid, imipenem, meropenem, clofazimine, viomycin, terizidone, TMC207, PA-824, OPC-7683, LL-3858 and SQ-109.

In another embodiment, one of said at least two agents is selected from the group consisting of isoniazid, rifampin, rifapentine, rifabutin, pyrazinamide, moxifloxacin, gatifloxacin, levofloxacin, ofloxacin, ciprofloxacin, and ethambutol.

In yet another embodiment, one of said at least two agents is pyrazinamide.

In yet another embodiment, one of said at least two agents is rifampin.

In yet another embodiment, one of said at least two agents is rifapentine.

In yet another embodiment, one of said at least two agents is PA-824.

In yet another embodiment, one of said at least two agents is OPC-67683

In yet another embodiment, one of said at least two agents is TMC-207.

In yet another embodiment, one of said at least two agents is selected from the group consisting of moxifloxacin, gatifloxacin, levofloxacin, and ofloxacin.

In yet another embodiment one of said at least two agents is moxifloxacin.

In a specific embodiment, said at least two agents are pyrazinamide and rifampin.

In yet another specific embodiment, said at least two agents are pyrazinamide, rifampin and isoniazid.

In yet another specific embodiment, said at least two agents are pyrazinamide and rifapentine.

In yet another specific embodiment, said at least two agents are pyrazinamide, rifapentine, and isoniazid.

In a specific embodiment, said at least two agents are pyrazinamide and moxifloxacin.

In yet another specific embodiment, said at least two agents is pyrazinamide, moxifloxacin, and rifampin.

In yet another specific embodiment, said at least two agents are pyrazinamide, moxifloxacin, and rifapentine

In yet another specific embodiment, said at least two agents are PA-824 and pyrazinamide.

In yet another specific embodiment, said at least two agents are PA-824, pyrazinamide and an agent selected from the group consisting of streptomycin, kanamycin, amikacin and capreomycin.

In yet another specific embodiment, said at least two agents are PA-824 and moxifloxacin.

In yet another specific embodiment, said at least two agents are PA-824, moxifloxacin and an agent selected from the group consisting of streptomycin, kanamycin, amikacin and capreomycin.

In yet another specific embodiment, said at least two agents are OPC-67683 and pyrazinamide.

In yet another specific embodiment, said at least two agents are OPC-67683, pyrazinamide and an agent selected from the group consisting of streptomycin, kanamycin, amikadn and capreomycin.

In yet another specific embodiment, said at least two agents are OPC-67683 and moxifloxacin.

In yet another specific embodiment, said at least two agents are OPC-67683, moxifloxacin and an agent selected from the group consisting of streptomycin, kanamycin, amikacin and capreomycin

In yet another specific embodiment, said at least two agents are TMC-207 and pyrazinamide.

In yet another specific embodiment, said at least two agents are TMC-207, pyrazinamide and moxifloxacin or isoniazid.

It was further found that a synergistic effect is observed when the compound of formula (I) of the invention is administered in combination with at least one agent useful in the treatment of tuberculosis, for example rifampin, during the continuation (sterilization) phase of treatment (after the initial phase of treatment) when non-multiplying persisting bacteria are prevalent. Such synergy is advantageous in that it may shorten the duration of treatment for TB. More significantly, the data below show that on administration of a compound of formula (I) in combination with at least two agents useful for the treatment of TB may shorten the duration of therapy for drug-susceptible TB, mono-drug-resistant TB and multi-drug resistant TB.

In one embodiment of the present invention, there is the use of a compound of formula (I) or a pharmaceutically acceptable salt thereof in combination with at least one anti-tuberculin agent in the manufacture of a medicament for treating tuberculorsis after a subject has undergone an initial phase of treatment, for tuberculosis.

In another embodiment, the at least one agent useful in the treatment of tuberculosis selected from the group consisting of rifampin, rifapentine, rifabutin, pyrazinamide, ethambutol, streptomycin, kanamycin, amikacin, moxifloxacin, gatifloxacin, levofloxadn, ofloxacin, ciprofloxacin, capreomycin, ethionamide, cycloserine, para-aminosalicylic acid, thiacetazone, clarithromycin, amoxicillin-clavulanic acid, imipenem, meropenem, clofazimine, viomycin, terizidone, TMC207, PA-824, OPC-7683, LL-3858 and SQ-109.

In yet another embodiment, said at least one agent is selected from the group consisting of rifampin, rifapentine, rifabutin, pyrazinamide, moxifloxacin, gatifloxacin, levofloxacin, ofloxacin, and ethambutol.

In a specific embodiment, said at least one agent is pyrazinamide.

In a specific embodiment, said at least one agent is rifampin.

In a specific embodiment, said at least one agent is rifapentine.

In a specific embodiment, said at least one agent is PA-824.

In a specific embodiment, said at least one agent is OPC-7683.

In a specific embodiment, said at least one agent is TMC-207.

In a specific embodiment, said at least one agent is selected from the group consisting of moxifloxacin, gatifloxacin, levofloxacin, and ofloxacin.

In a specific embodiment, said at least one agent is moxifloxacin. The uses and compositions of the invention are particularly effective against tuberculosis including active tuberculosis and latent tuberculosis. In one example, the active tuberculosis comprises drug-sensitive tuberculosis, mono-drug-resistant tuberculosis, multi-drug-resistant tuberculosis and extensively drug-resistant tuberculosis. In another example, there is disclosed a method to completely eradicate drug-sensitive tuberculosis, mono-drug resistant, multi-drug-resistant tuberculosis, and extensively drug-resistant tuberculosis (XDR) on completion of the treatment.

In addition, the uses and composition of the invention may be used in conjunction with diagnostic tests to identify the tuberculosis in the mammal which are known to those so skilled in the art. For example, the uses and compositions of the invention may be used in conjunction with the so-called line-probe assay (Haln Life-science GmbH) which may be used to identify genes linked with resistance to rifampin and isoniazid to indicate multi-drug-resistant tuberculosis and/or extensively drug-resistant tuberculosis. Other assays may also be used in conjunction with the uses and compositions of the invention, which are known to those so skilled in the art.

In another embodiment, the uses and compositions of the invention are particularly effective against tuberculosis caused by a *Mycobacterium* infection selected from the group consisting of *Mycobacterium tuberculosis, Mycobacterium bovis* or other related mycobacterial species, which would be known by one skilled in the art. In one example, the present invention provides uses and compositions to prevent relapse of the *Mycobacterium* nfection after completion of the treatment.

Such combination may be for simultaneous, separate or sequential use. In one embodiment, the at least two agents (or the at least one agent) useful for the treatment of tuberculosis are administered prior to administration of the compound of formula (I) of the invention. In another embodiment, the at least two agents (or the at least one agent) useful for the treatment of tuberculosis are administered after administration of the compound of formula (I) of the invention. In another embodiment, the at least two agents (or the at least one agent) useful for the treatment of tuberculosis are administered at about the same time as administration of the compound of formula (I) of the invention.

Separate administration of each compound, at different times and by different routes, in some cases would be advantageous. Thus, the components in the combination i.e. the compound of formula (I) of the invention and the at least two agents (or the at least one agent) in the treatment of tuberculosis need not be necessarily administered at essentially the same time or In any order. The administration can be so timed that the peak pharmacokinetic effect of one compound coincides with the peak pharmacokinetic effect of the other.

All the active ingredients can be formulated into separate or individual dosage forms which can be co-administered one after the other. Another option is that if the route of administration is the same (e.g. oral) two or more of the active compounds can be formulated into a single form for co-administration, both methods of co-administration, however, being part of the same therapeutic treatment or regimen.

Preferred agents useful for the treatment of tuberculosis may be as follows: isoniazid, rifampin, rifapentine, rifabutin, pyrazinamide, ethambutol, streptomycin, kanamycin, amikacin, moxifloxacin, gatifloxacin, levofloxacin, ofloxacin, ciprofloxacin, capreomycin, ethionamide, cycloserine, para-aminosalicylic acid, thiacetazone, clarithromycin, amoxicillin-clavulanic acid, imipenem, meropenem, clofazimine, viomycin, terizidone, TMC207, PA-824, OPC-7683, LL-3858 and SQ-109. The agents useful for the treatment of tuberculosis can be used in the present invention in.a variety of forms, including acid form, salt form, racemates, enantiomers, solvates, and tautomers. The agents useful for the treatment of tuberculosis may be administered by any route useful to administer said agents, which are known to those of skill in the art.

The invention also relates to compositions of the invention which comprise (i) a therapeutically effective amount of a compound of formula (I) or a pharmaceutically acceptable salt thereof, (ii) at least two agents useful in the treatment of tuberculosis and (iii) a pharmaceutically acceptable carriers or vehicles (hereinafter "the compositions of the invention").

Compositions of the invention that are suitable for administration to a patient in need thereof (e.g., a human) are also referred to herein as "pharmaceutical compositions of the invention."

The pharmaceutical compositions of the invention may be in any form suitable for administration to a patient. For example, the pharmaceutical compositions of the invention may be in a form suitable for oral administration such as a tablet, capsule, pill, powder, sustained release formulations, solution, and suspension; for parenteral injection as a sterile solution, suspension or emulsion; for topical administration as an ointment or cream; or for rectal administration as a suppository. The pharmaceutical compositions of the invention may be in unit dosage forms suitable for single administration of precise dosages.

Exemplary parenteral administration forms include solutions or suspensions of active compounds in sterile aqueous solutions, for example, aqueous propylene glycol or dextrose solutions. Such dosage forms can be suitably buffered, if desired.

In one embodiment, the pharmaceutical compositions of the invention may be in the form of an oral dosage form. Non-limiting examples of oral dosage forms include such as, e.g., chewable tablets, capsules, pills, lozenges, troches, sachets, powders, syrups, elixirs, solutions and suspensions, and the like, in accordance with standard pharmaceutical practice. In another embodiment, the pharmaceutical compositions of the invention can also be delivered directly to a patient's gastrointestinal tract through a nasogastric tube.

The compound of formula (I) of the invention will be present in the pharmaceutical composition of the invention in an amount sufficient to provide the desired dosage amount in the range described herein. The proportional ratio of compound of formula (I) of the invention to excipients will naturally depend on the chemical nature, solubility and stability of the active ingredients, as well as the dosage form contemplated. Typically, pharmaceutical compositions of the present invention can contain about 20% to about 99% of the compound of formula (I) of the invention by weight.

In one embodiment, the compound of formula (I) and pharmaceutically acceptable salts thereof is administered between about 10 mg to about 2000 mgln yet another embodiment, the compound of formula (I) and pharmaceutically acceptable salts thereof is administered between about 25 mg to about 1000 mg.

In yet another embodiment, the compound of formula (I) and pharmaceutically acceptable salts thereof is administered between about 50 mg to about 500 mg.

In yet another embodiment, the compound of formula (I) and pharmaceutically acceptable salts thereof is administered between about 100 mg to about 500 mg.

Techniques for formulation and administration of the compound of formula (I) of the instant invention and the compositions of the invention the can be found in Remington: the Science and Practice of Pharmacy, 19th ed., Mack Pub. Co., Easton, Pa. (1995).

The term "excipient" means an inert material that is combined with the compound of formula (I) to produce a pharmaceutical composition or oral drug dosage form.

The term "pharmaceutically acceptable excipient" means that the excipient must be compatible with other ingredients of the composition, and not deleterious to the recipient thereof. The pharmaceutically acceptable excipients are chosen on the basis of the intended dosage form.

The tablets, pills, capsules, and the like may contain excipients selected from binders such as polyvinylpyrrolidone, hydroxypropylmethyl cellulose (HPMC), hydroxypropylcellulose (HPC), sucrose, gelatin, acacia, gum tragacanth, or corn starch; fillers such as microcrystalline cellulose, lactose, sodium citrate, calcium carbonate, dibasic calcium phosphate, glycine and starch; disintegrants such as corn starch, potato starch, alginic acid, sodium starch glycolate, croscarmellose sodium and certain complex silicates; lubricants such as magnesium stearate, sodium lauryl sulfate and talc; and sweeteners such as sucrose lactose or saccharin. When a dosage unit form is a capsule, it may contain, in addition to materials of the above type, a liquid carrier such as a fatty oil. Various other materials may be present as coatings or to modify the physical form of the dosage unit. For instance, tablets may be coated with shellac, sugar or both.

In the case of pediatric oral suspensions and sachets, these excipients may comprise suspending aids such as xantham gum or hydroxypropylmethylcellulose, glidants such as colloidal silica, diluents and bulking agents such as silicon dioxide, flavors such as bubble gum, orange, banana, raspberry and golden syrup or mixtures thereof, sweeteners such as aspartame or sugar, and stabilizers such as succinic acid. Powder or granular formulations, such as pediatric suspension formulations and sachets, may be manufactured using techniques which are generally conventional in the field of manufacture of pharmaceutical formulations and in the manufacture of dry formulations for reconstitution into such suspensions. For example a suitable technique is that of mixing dry powdered or granulated ingredients.

Typically, an effective daily dose (i.e., total dosage over about 24 hours) of the compound of formula (I) of the invention for adults is about 10 mg to about 2000 mg; about 25 mg to about 1000 mg; about 50 mg to about 500 mg; and 100 mg to about 500 mg with or without food. In some cases, it may be necessary to use dosages outside these limits.

A daily dosage of the compound of formula (I) of the invention is usually administered from 1 to 4 times daily in equal doses.

In one embodiment, a single dose of compound of formula (I) of the invention is administered per day (i.e., in about 24 hour intervals) (i.e., QD); in another embodiment, two doses of compound of formula (I) of the invention are administered per day (i.e., BID); in another embodiment, three doses of compound of formula (I) of the invention are administered per day (i.e., TID); and in another embodiment, four doses of compound of formula (I) of the invention are administered per day (i.e., (QID); in another embodiment a single dose of compound of formula (I) of the invention is administered every other day (i.e., in about 48 hour intervals), in another embodiment a single dose of compound of formula (I) of the invention is administered twice per week; in another embodiment a single dose of compound of formula (I) of the invention is administered thrice per week.

In one embodiment, the effective dose of the compound of formula (I) of the invention is administered BID in about 12 hour intervals.

In another embodiment, the effective dose of the compound of formula (I) of the invention is administered TID in about 8 hour intervals.

In another embodiment, the effective dose of the compound of formula (I) of the invention for is administered QID in about 6 hour intervals.

In one embodiment, an effective dose of the compound of formula (I) of the invention is about 25 mg to about 1000 mg which is administered BID In about 12 hour intervals.

Oral administration is preferred.

The pharmaceutical composition of the invention in a fixed dose combination comprising a compound of formula (I) of the invention and two agents useful for the treatment of tuberculosis and pharmaceutically acceptable carriers can be prepared by conventional methods in the art. For e.g., a tablet form of the combination can be prepared by any one of skill in the art

Some examples of the present invention are combinations and the pharmaceutical compositions which encompass the following non-limiting mixtures:
a) a compound of formula (I) or a pharmaceutically acceptable salt thereof and pyrazinamide;
b) a compound of formula (I) or a pharmaceutically acceptable salt thereof and rifampin;
c) a compound of formula (I) or a pharmaceutically acceptable salt thereof and rifapentine;
d) a compound of formula (I) or a pharmaceutically acceptable salt thereof, and PA-824;
e) a compound of formula (I) or a pharmaceutically acceptable salt thereof, and TMC-207;
f) a compound of formula (I) or a pharmaceutically acceptable salt thereof, and an agent selected from the group consisting of moxifloxacin, gatifloxacin, levofloxacin, ofloxacin moxifloxacin;
g) a compound of formula (I) or a pharmaceutically acceptable salt thereof, isoniazid and rifampin;
h) a compound of formula (I) or a pharmaceutically acceptable salt thereof, isoniazid, rifampin and pyrazinamide; and
i) a compound of formula (I) or a pharmaceutically acceptable salt thereof, rifampin and pyrazinamide.

Other examples of the present invention are combinations and the pharmaceutical compositions that encompass the following non-limiting mixtures,
j) about 100 mg to about 500 mg of a compound of formula (I) or a pharmaceutically acceptable salt thereof and about 600 mg of rifampin;
k) about 100 mg to about 500 mg of a compound of formula (I) or a pharmaceutically acceptable salt thereof and about 300 mg of isoniazid;
l) about 100 mg to about 500 mg of a compound of formula (I) or a pharmaceutically acceptable salt thereof and about 300 mg of isoniazid and about 600 mg of rifampin;
m) about 100 mg to about 500 mg of a compound of formula (I) or a pharmaceutically acceptable salt thereof, about 300 mg of isoniazid, about 600 mg of rifampin and about 20-25 mg/kg to about 50-70 mg/kg of pyrazinamide;
n) about 100 mg to about 500 mg of a compound of formula (I) or a pharmaceutically acceptable salt thereof and about 10 mg/kg to about 20 mg/kg of rifampin;
o) about 100 mg to about 500 mg of a compound of formula (I) or a pharmaceutically acceptable salt thereof and about 10-15 mg/kg to about 20-30 mg/kg of isoniazid;
p) about 100 mg to about 500 mg of a compound of formula (I) or a pharmaceutically acceptable salt thereof and about 10-15 mg/kg to about 20-30 mg/kg of isoniazid and about 10 mg/kg to about 20 mg/kg of rifampin;
q) about 100 mg to about 500 mg of a compound of formula (I) or a pharmaceutically acceptable salt thereof, about 10-15 mg/kg to about 20-30 mg/kg of isoniazid, about 10 mg/kg to about 20 mg/kg of rifampin and about 15-30 mg/kg to about 50 mg/kg pyrazinamide;
r) about 50 mg to about 250 mg of a compound of formula (I) or a pharmaceutically acceptable salt thereof, about 75 mg isoniazid, about 150 mg of rifampin and about 400 mg of pyrazinamide; and
s) about 25 mg to about 250 mg of a compound of formula (I) or a pharmaceutically acceptable salt thereof, and about 300 mg of rifampin.

The compounds of formula (I) of the present invention are readily prepared according to synthetic methods familiar to those skilled in the art. The compound of formula (I) of the invention can be prepared in a manner similar to that described for the preparation of Example 1 described in the Examples section in U.S. Patent No. 5,880,118. In addition, the compound of formula (I) can be prepared by the processes set forth in international Publication WO97/37980 and WO99/24393, both of which are herein incorporated by reference.

Another example of the preparation of (S)-N-[[3-[3-fluoro-4-(4-thiomorpholinyl)phenyl]-2-oxo-5-oxazolidinyl]methyl]acetamide is as follows:

Scheme 1 illustrates a general synthetic sequence for preparing compounds of the present invention.

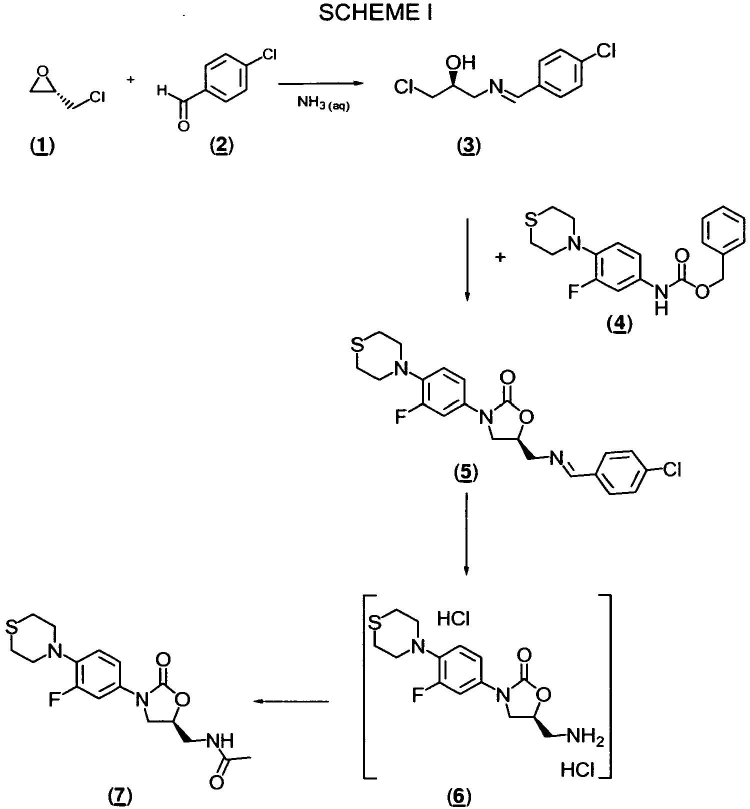

Scheme 1 illustrates a method of synthesizing compounds of formula (I) of the invention in a multistep synthesis via a compound of formula 6. Referring to Scheme I, the synthesis begins with the formation of intermediate (3) by reacting (*S*)-epichlorohydrin (1) with a mixture of the appropriately substituted benzaldehyde derivative (2) (preferably 0.5 to 2 eq, most preferably 1 eq) and aqueous ammonia (preferably 0.5 to 3 eq, most preferably 1.5 eq). The reaction is best performed in both protic and aprotic non-nucleophilic and inert solvents such as alcohols (including C₁-C₆ branched and linear alcohols and polyols), ethers (including MTBE, THF, and other C₁₋C₆ linear, branched and cyclic ethers) as well as chlorinated solvents such as methylene chloride. MTBE is a preferred solvent. Temperatures in a range from about 15 to about 60°C are preferred, most preferably between 30 to 50°C. After extractive isolation and concentration, the imine moiety (3) is obtained. It is then crystallized from a second liquid phase, in the presence of non-polar hydrocarbon solvents such as, but not limited to, alkanes, mixtures of alkanes (hexane, heptane, octane, iso-octane and commercially available alkane mixtures), optionally in the presence of aprotic polar solvents, preferably ethereal solvents such as MTBE or aromatic solvents such as toluene or chlorinated solvents such as methylene chloride or mixtures thereof. Preferred solvents are a mixture of MTBE and heptane or a mixture of toluene and heptane. The crystallization process can be conducted at a temperature in a range from ambient temperature (about 18-25°C) to about 55°C, preferably in a range of 30 to 50°C, more preferably in a range of 38 to 45°C. This crystallization provides surprisingly high yield and affords significantly improved enantiomeric purity after isolation by filtration. (S)-epichlorohydrin (1) and benzaldehyde derivative (2) are commercially available or can be made by methods well known to those skilled in the art.

The substituted imine moiety (3) is coupled with carbamate (4) (which is known to those skilled in the art, for example see J. Med. Chem., 1996, 39, (3), 680-685 and also Example 2 below, (preferably 1 to 3 eq, most preferably 1.5 to 2 eq)) to provide the corresponding (S)-oxazolidinone imine (5). The reaction is carried out preferably at a temperature in a range from ambient temperature to about 65°C in the presence of a base with pKa greater than 12, preferably a tertiary alkoxide base, most preferably lithium tert-butoxide and an aprotic non-nucleophilic solvent (preferably DMF, DMAc, acetonitrile, C₁-C₆ linear, branched and cyclic ethers and/or chlorinated solvents and/ or mixtures of these solvents, most preferably MTBE or methylene chloride). Most preferably, the temperature is from about 30-60°C and the reaction time is 2 to 24 hours. Preferably, the (S)-oxazolidinone imine (5), after an aqueous extractive workup, is isolated by filtration from a 1:1 mixture of an ether (including MTBE, THF, and other C₁-C₆linear, branched and cyclic ethers) and water, most preferably MTBE. Alternatively, (5) is isolated after an aqueous extractive workup, by filtration or crystallization from an alcohol (including C₁-C₆linear, branched alcohols and polyols); most preferably isopropanol. Hydrolysis of compound (5) with an aqueous acidic solution provides compound (6) and subsequent acylation provides crude compound (7). Compound (5) is best hydrolyzed with a mixture of water and a strong acid such as hydrochloric acid and the substituted benzaldehyde byproduct is removed by extraction with a water immiscible organic solvent (preferably toluene, MTBE, methylene chloride or ethyl acetate), most preferably ethyl acetate. The resulting aqueous solution of amine hydrochloride (6) is preferably acylated with acetic anhydride, preferably in the presence of water and a water-immiscible organic solvent (most preferably methylene chloride). The conversion of amine hydrochloride (6) to compound (7) is well known in the literature. (See Brickner, S.J. et. al. J. Med. Chem. 1996 39 (3) 673-679, US Patent 5,837,870, US 5,688,792).

The examples provided below further illustrate and exemplify the compounds of formula (I) of the invention, compositions of the invention and methods of using the compound of the invention. It is to be understood that the scope of the present invention is not limited in any way by the scope of the following examples and preparations.

### Example 1

### Preparation of (S)-1-chloro-3-[(4-chloro-E-benzylidene)-amino]-propan-2-ol

### Method A

A 5L three neck round bottom flask equipped with a mechanical stirrer, thermocouple, reflux condenser and heating mantle is charged with 4-chlorobenzaldehyde (351.0 g, 2.5 mol, 1.0 eq.). MTBE (1.5 L) is then charged into the round bottom to give a homogeneous solution. Aqueous ammonia (28 wt%, 252.98 mL, 3.75 mol, 1.5 eq.) is added in a single portion resulting in a white precipitate that turned into a thin slurry within 15 minutes of stirring. (S)-(+)-epichlorohydrin (> 99 % ee, 196.0 mL, 2.5 mol, 1.0 eq.) is then slowly charged into the vessel. After 40 minutes, the contents are then slowly heated to 43°C. The reaction is stirred at 40°C for 18 hours at which time 8.4% area of epichorohydrin remained by GC. Upon cooling, the reaction mixture is transferred to a separatory funnel and the layers are separated. The lower aqueous layer is discarded. The organic layer is transferred to a 3L round bottom flask, concentrated *in vacuo* to about half the volume (800-900 mL) at which time iso-octane is slowly added from a feed tube (-750 mL) until cloudiness is observed. The biphasic mixture is seeded with -4 mgs of the title compound. The reaction is cooled with an ice bath for 45 minutes while stirring. The precipitate is collected and rinsed with cold *iso-*octane (500 mL). The solid is dried for 18 hours at 50°C under vacuum to give 345.19 g (59% yield) of the title compound as a while solid. GC assay: 100%, 99.7% ee by Chiral SFC). GC (conditions: column - 30 meter HP-1, 0.25 mm ID and 0.25 micron film and 15 psi head pressure, 1.0 µl injection size; Tᵢₙᵢ = 70°C, ramp of 20°C/min) T_{R} (epichlorohydrin) = 2.4 min, T_{R} (4-chlorobenzaldehyde) = 4.8 min and T_{R} (title compound) = 9.7 min; HPLC conditions: Chiralpak AD-H 250 mm X 4.6 mm column, eluting with 70% CO₂/ 30% MeOH at 3.0 mL/min, detecting at 255 nm. T_{R} [title compound] = 3.9 min; T_{R} (enantiomer of title compound) = 2.8 min; ¹H NMR (400 MHz, CDCl₃) δ 3.69 (bs, 2 H), 3.80 (m, 2 H), 4.15 (s, 1 H), 7.41 (d, J = 8 Hz, 2 H), 7.69 (d, J = 8 Hz, 2 H), 8.33 (s, 1 H); ¹³C NMR (CDCl₃) δ 47.05, 63.09, 70.82, 128.93, 129.39, 134.08, 137.07, 162.30; IR (KBr Pellet) 1630 cm⁻¹.

### Method B

A 5L three neck round bottom flask equipped with a mechanical stirrer, thermocouple, reflux condenser and heating mantle is charged with 4-chlorobenzaldehyde (375 g, 2.67 mol, 1.0 eq.). Methanol (0.75 L) is added to give a homogeneous solution after warming from 10 to 23°C. Aqueous ammonia (28.4 wt%, 264 mL, 3.95 mol, 1.5 eq.) is added in a single portion resulting in a biphasic solution forming after stirring for 15 minutes at 23 to 26°C. (S)-(+)-epichlorohydrin (99.3 % ee, 207 mL, 2.64 mol, 1.0 eq.) is then added in one portion. The reaction mixture is stirred at 23-24°C for 18 hours, then warmed to 40 to 45°C and stirred for 2.5 hours at which time 0.26% area of (S)-epichorohydrin remains by GC (GC conditions, 0.050 mL reaction mixture in 1 mL acetonitrile, inject 1 microliter; 15 M DB-1 column, 0.25 mm ID and 0.25 micron film and 15 psi head pressure, 1.0 µl injection size; Tᵢₙᵢ = 38°C, ramp of 10°C/min) T_{R} (epichlorohydrin) = 1.1 min, T_{R} (4-chlorobenzaldehyde) = 6.9 min and T_{R} (title compound) = 16.0 min). The mixture is concentrated *in vacuo* to a total volume of 1250 mL. Toluene (250 mL) is added and the mixture concentrated *in vacuo* to a total volume of 1250 mL. Toluene (250 mL) is added and the mixture concentrated *in vacuo* to a total volume of 1145 mL. Toluene (355 mL) is added and the mixture concentrated *in vacuo* to a total volume of 900 mL. Toluene (600 mL) is added and the mixture concentrated *in vacuo* to a total volume of 1120 mL. While maintaining 45 to 50°C, heptane (1500 mL) is added. The resulting biphasic solution is cooled to 45°C and seeded. The mixture is then further cooled to 38°C over 1/2 hour while seeding after every 1 degree of cooling. The mixture is then further allowed to slowly cool to 23°C over 16 hours. The white crystals are then collected by vacuum filtration and washed with room temperature heptane (180 mL). The product is dried in a nitrogen stream to give the title compound (431.57 g, 70.4%). HPLC 95 area% [Kromasil 150 mm X 4.6 mm column, 254 nm, flow rate 1.5 mL/ min; A = 1000 mL water + 0.52 mL trifluoroacetic acid + 1.20 mL triethylamine; B = acetonitrile; Isocratic 47: 53 A: B for 5 min then gradient to 100% B over 5 min T_{R} [title compound] = 2.1 min; T_{R} (4-chlorobenzaldehyde) = 2.3 min]; 99.72% ee by Chiral SFC. Chiral HPLC conditions: Chiralpak AD-H 250 mm X 4.6 mm column, eluting with 70% CO₂/ 30% MeOH at 3.0 mL/min, detecting at 255 nm. T_{R} [title compound] = 3.9 min; T_{R} (enantiomer of title compound) = 2.8 min. ¹H NMR (400 MHz, CDCl₃) δ 3.69 (bs, 2 H), 3.80 (m, 2 H), 4.15 (s, 1 H), 7.41 (d, J = 8 Hz, 2 H), 7.69 (d, J = 8 Hz, 2 H), 8.33 (s, 1 H); ¹³C NMR (CDCl₃) δ 47.05, 63.09, 70.82, 128.93, 129.39, 134.08,137.07, 162.30.

### Method C

A 5L three neck round bottom flask equipped with a mechanical stirrer, thermocouple, reflux condenser and heating mantle is charged with 4-chlorobenzaldehyde (375 g, 2.67 mol, 1.0 eq.). MTBE (1.50 L) is then added to give a homogeneous solution after warming from 9 to 24°C. Aqueous ammonia (28.4 wt%, 265 mL, 3.97 mol, 1.5 eq.) is added in a single portion resulting in a biphasic solution forming after stirring for 15 minutes at 23 to 26°C. (S)-(+)-epichlorohydrin (99.3 % ee, 209 mL, 2.67 mol, 1.0 eq.) is then added in one portion. The reaction mixture is stirred at 23-24°C for 3 days. The phases are separated and the upper phase concentrated under atmospheric pressure from 2000 to 1000 mL total volume (boiling point 58 to 67°C). While maintaining 45 to 50°C, heptane (1700 mL) is added. The resulting biphasic solution is cooled to 45°C and seeded. The mixture is then further cooled to 38°C over 1/2 hour while seeding after every 1 degree of cooling. The mixture is then further allowed to slowly cool to 23°C over 1 hour. The snow white heavy crystals are then collected by vacuum filtration and washed with room temperature heptane (180 mL). The product is dried in a nitrogen stream to give the title compound (462.43 g, 74.7%). HPLC 94 area% [Kromasil 150 mm X 4.6 mm column, 254 nm, flow rate 1.5 mL/ min; A = 1000 mL water + 0.52 mL trifluoroacetic acid + 1.20 mL triethylamine; B = acetonitrile; Isocratic 47: 53 A: B for 5 min then gradient to 100% B over 5 min. T_{R} [title compound] = 2.1 min; T_{R} (4-chlorobenzaldehyde) = 2.3 min]; 99.92% ee by Chiral SFC. Chiral HPLC conditions: Chiralpak AD-H 250 mm X 4.6 mm column, eluting with 70% CO₂/ 30% MeOH at 3.0 mL/min, detecting at 255 nm. T_{R} [title compound] = 3.9 min; T_{R} (enantiomer of title compound) = 2.8 min; ¹H NMR (400 MHz, CDCl₃) δ 3.69 (bs, 2 H), 3.80 (m, 2 H), 4.15 (s, 1 H), 7.41 (d, J = 8 Hz, 2 H), 7.69 (d, J = 8 Hz, 2 H), 8.33 (s, 1 H); ¹³C NMR (CDCl₃) δ 47.05, 63.09, 70.82, 128.93, 129.39, 134.08, 137.07, 162.30.

### Example 2

### Preparation of (3-fluoro-4-morpholin-4-yl-phenyl)-carbamic acid benzyl ester

The title compound can be prepared according to the method described in *J*. *Med. Chem*., **1996**, *39*, (3), 680-685 and depicted in SCHEME II.

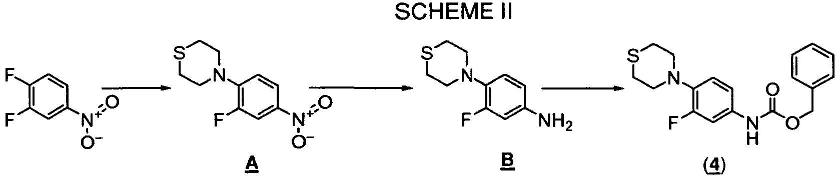

Additional methods for the conversion of intermediate A to 3-fluoro-4-thiomorpholin-4-ylaniline (B) are provided.

### Method A

4-(2-Fluoro-4-nitrophenyl)thiomorpholine (A, 250 g, 1.03 mole) was charged into a mixture of dioxane (1400 mL), EtOH (1000 mL) and water (600 mL) in a 5000 mL three neck round bottom flask equipped with a mechanical stirrer. Into the stirred mixture was charged ammonium chloride (166 g, 3.1 moles) followed by iron powder (247 g, 4.25 moles), each in single portions. The reaction was warmed to reflux with vigorous stirring. The reaction was heated at reflux for a total of 16 hours and was then allowed to cool to room temperature. The dark mixture was diluted with EtOAc (800 mL), filtered through a pad of celite, and concentrated *in vacuo* to a pasty residue. The residue was partitioned between brine (1000 mL) and dichloromethane (750 mL). One filtration through celite removed particulates that were interfering with phase separation. The aqueous layer was then extracted with additional dichloromethane (750 mL). The combined organic layers were dried over anhydrous potassium carbonate and concentrated *in vacuo* to give 225 g of a dark solid. This crude material was dissolved in dichloromethane (1000 mL), treated with 200 g silica gel (230-400 mesh) and the mixture was concentrated to dryness. The plug was filtered over 500 g silica gel (230-400 mesh, packed as a slurry with 20% EtOAc/hexane) eluting with 20-30% EtOAc/hexane while collecting 1000 mL fractions. Fractions 3-11 were combined and concentrated to give 3-fluoro-4-thiomorpholin-4-ylaniline (B, 232 g, 106% yield) as an off-white solid. ¹H NMR indicated the desired material along with trace residual solvents to account for the greater than theoretical recovery. ¹H NMR (400 MHz, CDCl₃): δ 2.8 (m, 4H), 3.2 (m, 4H), 3.6 (s, 2H), 6.4 (m, 2H), 6.8 (m, 1H).

### Method B

A 2000 mL Parr shaker flask was charged with 5% sulfided palladium on carbon (Johnson Matthey type A103038-5, 18 g) and 4-(2-fluoro-4-nitrophenyl)thiomorpholine (A, 60 g, 0.25 mole). The mixture was suspended in MeOH (1050 mL) and the reaction was hydrogenated at 50 PSI for 7 h. The catalyst was removed by filtration through celite and the filter cake was washed well with fresh MeOH. The clear gray filtrate was concentrated *in vacuo* to give 3-fluoro-4-thiomorpholin-4-ylaniline (B, 51.3 g, 98% yield) as a gray solid. ¹H NMR (400 MHz, CDCl₃): δ 2.8 (m, 4H), 3.2 (m, 4H), 3.6 (s, 2H), 6.4 (m, 2H), 6.8 (m, 1 H).

### Example 3

### Preparation of (5S)-5-4{[(4-chlorobenzylidene)amino]methyl}-3-(3-fluoro-4-thiomorpholin-4-ylphenyl)-1,3-oxazolidin-2-one

The title compound in Example 2 (194 g, 0.56 mole), and the title compound of Example 1 (195 g, 0.84 mole), and lithium *tert*-butoxide (116 g, 1.4 mole) were charged into a 3000 mL three neck round bottom flask under nitrogen. The reactants were slurried with methyl *tert*-butyl ether (1200 mL) and the mixture was warmed to 56°C and stirred for 2 h as a yellow solid gradually formed. The reaction was cooled to room temperature, and diluted with 1200 mL water. The mixture was then stirred vigorously over 60 min as the solid changed from dark yellow to a more pale yellow solid. The mixture was cooled to 10°C, filtered, and the filter cake was washed with ice cold methyl *tert*-butyl ether (450 mL). The resulting light yellow solid was dried in air for 30 min, then placed in a vacuum oven and dried at 40°C overnight to afford the title compound (243 g, 99% yield). ¹H NMR (400 MHz, CDCl₃): δ 2.8 (m, 4H), 3.2 (m, 4H), 3.9 (m, 2H), 4.1 (m, 2H), 5.0 (m, 1H), 6.9 (m, 1 H), 7.2 (m, 1H), 7.4 (m, 3H), 7.6 (m, 2H), 8.4 (s, 1H).

### Example 4

### Preparation of N-{[(5S)-3-(3-fluoro-4-thiomorpholin-4-ylphenyl)-2-oxo-1,3-oxazolidin-5-yl]methyl}acetamide

The title compound in Example 3 (243 g, 0.56 mole) was combined with EtOAc (1300 mL) and water (1300 mL) in a 5000 mL three neck round bottom flask equipped with a mechanical stirrer. The mixture was treated drop-wise with 12N HCl (140 mL, 1.68 moles) and the mixture was stirred vigorously for 1 hour at room temperature. The layers were separated and the aqueous layer was washed with EtOAc (1 x 500 mL). The resulting aqueous solution containing (*S*)-5-(aminomethyl)-3-(3-fluoro-4-thiomorpholinophenyl)oxazolidin-2-one hydrochloride was combined with a mixture of dichloromethane (1800 mL) and MeOH (120 mL), and the vigorously stirred mixture was charged with acetic anhydride (132 mL, 1.4 mole) in one portion and subsequently treated drop-wise with 10 N NaOH (200 mL, 2.0 mole) over 15 min. An extremely thick reaction mixture resulted from addition of the base, which gradually thinned as the pH rose and the acylation rapidly progressed. The reaction was stirred vigorously for 1 hour after the mixture resolved to two phases. At that time, 10 M NaOH (160 mL, 1.6 mole) was added drop-wise to the mixture until the pH was stable at 7. The layers were separated, the aqueous layer was extracted with dichloromethane (250 mL), and the combined organic layers were dried over anhydrous potassium carbonate. The volatiles were removed *in vacuo* to give an off-white solid which was titrated with methyl *tert-*butyl ether (250 mL), collected, and dried *in vacuo* to give title compound (5) (186.1 g, 94% yield) as a fine white solid with greater than 98% HPLC purity (retention time = 3.93 minutes, HPLC conditions reported below).

The crude solid was dissolved in warm 6% methanol in dichloromethane (1250 mL) in a 5000 mL three neck round bottom flask equipped with a mechanical stirrer. The solution was warmed to reflux, diluted by the portion-wise (500 mL) addition of 2500 mL isopropanol (IPA), and, in order to maintain reflux, the temperature was ramped to 50-70°C. On completion of this addition of IPA, the reflux condenser was replaced with a short-path distillation head and distillation was continued into a cooled flask. During distillation, a 500 mL portion of fresh IPA was added after 500 mL of distillate was collected to maintain between 2000 and 2500 mL IPA present at all times. After this addition (internal flask temperature dropped to 60°C) the mixture became slightly cloudy and remained so for the balance of the distillation, becoming increasingly cloudy as the distillate temperature exceeded 70°C; particulate matter appeared as the distillate temperature exceeded 75°C. The temperature controller was ramped to 85°C and held there until the conclusion of the distillation. When the distillate was clearly isopropanol alone (82-83°C) the volume was reduced to 2500 mL hot IPA, the heating mantle was removed, stirring was discontinued, and the paddle was removed from the flask. The mixture was allowed to continue to crystallize as the flask cooled. The white crystalline solid was then collected by filtration, washed with methyl tert-butyl ether (250 mL), and dried *in vacuo* at 40°C to afford 180 g (91 % yield) of the title compound in greater than 99% HPLC purity (retention time = 3.93 minutes, HPLC conditions reported below). ¹H NMR (400 MHz, DMSO-d₆): δ 1.8 (s, 3H), 2.7 (m, 4H), 3.2 (m, 4H), 3.4 (m, 2H), 3.7 (m, 1H), 4.7 (m, 1H), 7.1 (m, 1 H), 7.15 (m, 1 H), 7.2 (m, 1 H), 8.2 (m, 1H). Mass Spec. C₁₆H₂₀FN₃O₃S: m/z 354.1 (M+1).

HPLC conditions for analyses mentioned in the text: HP Series 1100; Column: Symmetry C8 5uM 4.6 x 50 mm; Flow rate 1.2 mL/min; Solvent A: water with 0.1 % formic acid, Solvent B: acetonitrile with 0.1% formic acid; Injection volume = 10 uL of 1 mg/mL (acetonitrile); Gradient: Solvent B 0-100% over 7 minutes then 100% B for 1 minute; wavelength = 254 nm.

### BIOLOGICAL EXAMPLES

The following abbreviations are used in the following Examples and have the definitions indicated unless otherwise noted: CFU is colony forming unit; INH is isoniazid; RIF is rifampin; PZA is pyrazinamide; MXF is moxifloxacin, and LZD is linezolid.

The bacterial strain *Mycobacterium tuberculosis* H37Rv was passaged in mice, frozen in 1 mL aliquots and stored at -80°C before use. For each infection, an aliquot was thawed and sub-cultured in Middlebrook 7H9 broth supplemented with 10% oleic acid-albumin-dextrose-catalase (OADC) (Difco, Detroit, MI) and 0.05% Tween 80 (Sigma, St. Louis MO). Female BALB/c mice (Charles River, Wilmington, MA), aged four to six weeks were infected via aerosol using the Inhalation Exposure System (Glas-col Inc, Terre Haute, IN) and a log phase broth culture with an optical density of approximately 1.0 at 600 nm. Mice were randomized to treatment groups (5 mice per group per time point) after aerosol infection. Untreated mice were routinely sacrificed (i) on the day after infection to determine the number of CFU implanted in the lungs and (ii) on the day of treatment initiation to determine the pre-treatment CFU count.

The compound of formula (I) of the invention and LZD (obtained from Pfizer Inc. Ann Arbor, MI and Groton, CT.) used in the tests described below was suspended in a solution composed of 5% polyethylene glycol-200 (PEG-200) and 95% methylcellulose (0.5%) in distilled water. MXF was obtained from Bayer (Rolling Meadows, IL), PZA was obtained from Fisher, and INH and RIF were obtained from Sigma. Stock solutions were prepared weekly using distilled water. All antibiotic solutions were stored at 4°C.

Except where otherwise indicated, antibiotics were administered once daily, five days per week in 0.2 ml by gavage. Both oxazolidinone suspensions were sonicated briefly prior to use and shaken between doses. RIF was given 1 hour prior to administration of other drugs to avoid an adverse pharmacokinetic interaction.

### Example 5

### In vitro activity of the compound of formula (I)

The MIC of the compound of formula (I) and LZD was determined by the agar dilution method on Middlebrook 7H11 agar supplemented with 10% OADC (Becton-Dickinson, Sparks, MD). Plates containing serial two-fold concentrations of the compound of formula (I) and LZD ranging from 0.125 to 4 µg/mL were inoculated with approximately 5x10⁵ CFU of *Mycobacterium tuberculosis* H37Rv. CFU were counted after 28 days incubation at 37°C with 5% ambient CO₂. The MIC was defined as the lowest concentration to inhibit at least 99% of bacterial growth.

The MIC of the compound of formula (I) and LZD against *Mycobacterium tuberculosis* H37Rv was 0.25 µg/mL.

### Dose-ranging activity of the compound of formula (I) and LZD in an established infection model

Mice were infected by the aerosol route with 4.44 ± 0.04 log₁₀ CFU. Beginning 13 days after aerosol infection, when the mean lung CFU count was 7.49 ± 0.11 log₁₀ and the mean spleen weight was 105 ± 11 mg, control mice received one of the following treatments: INH at 25 mg/kg, LZD 25 mg/kg to 260 mg/kg (single dose and 130 mg/kg twice daily), and compound of formula (I) 25 mg/kg to 100 mg/kg doses. At the end of the treatment period, 28 days later, only 2 of 5 untreated mice remained alive. The mean spleen weight among those living mice had increased to 237 ± 16 mg.

In contrast, monotherapy with INH at 25 mg/kg prevented splenomegaly (mean spleen weight = 100 ± 1 mg) and death. A dose-dependent decrease in spleen weight relative to untreated controls was observed with increasing doses of LZD between 25 mg/kg (200 ± 47 mg) and 130 mg/kg (102 ± 5 mg). However, spleens from mice treated with LZD 260 mg/kg (whether as a single dose or 130 mg/kg twice daily) were larger than expected (190 ± 52 mg) and similar in size to spleens from mice treated with LZD 25 mg/kg/day. Because the number and size of lung lesions decreased with increasing daily doses of LZD including 260 mg/kg, the splenomegaly observed in mice receiving 260 mg/kg/day was not felt to be due to reduced anti-tuberculosis activity. Treatment with the compound of formula (I) prevented splenomegaly at all doses administered, including the 25 mg/kg dose (mean spleen weight = 113 ± 17 mg).

Surviving untreated mice experienced an increase in CFU counts to nearly 8 log₁₀. Treatment with INH reduced the mean lung CFU count to 5.62 log₁₀, for a log kill of 1.87 compared to the baseline value. All regimens including the compound of formula (I) resulted in a significant reduction in mean CFU count from baseline (p<0.01), starting with a 0.78 log₁₀ reduction with 25 mg/kg. At 50 mg/kg, the compound of formula (I) exhibited bactericidal activity (defined by the 2-log-reduction criterion) by reducing the mean CFU count to 5.28 log₁₀, for a log kill of 2.21, greater than that observed with INH. Although LZD also displayed dose-dependent activity, its activity was more limited than that of the compound of formula (I). Only at LZD doses ≥100 mg/kg was a significant reduction from the baseline CFU count demonstrated (p<0.01). Even at the highest dose tested, 260 mg/kg, LZD did not meet the 2-log-reduction criterion defining bactericidal activity, producing a log kill of only 1.46. The compound of formula (I) was significantly more active than LZD at each dose tested.
Treatment with the compound of formula (I) at 25 mg/kg resulted in a CFU count lower than that observed after treatment with LZD at 25 or 50 mg/kg (p<0.01) and not significantly different from that observed after treatment with LZD at 100 or 130 mg/kg. At 50 and 100 mg/kg, the compound of formula (I) was more active than any dose of LZD (p<0.001). Dividing the total daily dose of the compound of formula (I) and LZD into two daily doses had no clear effect (i.e., positive or negative) on activity (Table 1).

**Table 1**

| | Daily dosing regimen | Mean log₁₀ CFU count (± SD) on: | |
|---|---|---|---|
| Drug | | Day 0 | Day 28 |
| None | None | 7.49 ± 0.11 | 7.92 ± 0.03 |
| INH | 25 m/kg once | | 5.62 ± 0.20 |
| Compound of Formula (I) | 25 mg/kg once | | 6.71 ± 0.28 |
| | 25 mg/kg twice | | 5.51 ± 0.14 |
| | 50 mg/kg once | | 5.28 ± 0.53 |
| | 50 mg/kg twice | | 4.89 ± 0.27 |
| | 100 mg/kg once | | 5.07 ± 0.14 |
| LZD | 25 mg/kg once | | 7.55 ± 0.24 |
| | 50 mg/kg once | | 7.33 ± 0.11 |
| | 100 mg/kg once | | 6.75 ± 0.14 |
| | 130 mg/kg once | | 6.57 ± 0.13 |
| | 130 mg/kg twice | | 6.02 ± 0.17 |
| | 260 mg/kg once | | 6.03 ± 0.12 |

Hence, the compound of formula (I) demonstrated that it is significantly more active than the human-equivalent dose (i.e. 100 mg/kg) of LZD, the only clinically available oxazolidinone, which is being used off-label for the treatment of MDR- and XDR-TB.

### Pharmacokinetics of the compound of formula (I)

To determine the single-dose and steady-state pharmacokinetic profiles of the compound of formula (I) and LZD in the murine model, a sub-study was nested in the above dose-ranging study using mice treated with the compound of formula (I) at 100 mg/kg once-daily or LZD at 130 mg/kg once- or twice-daily. Three mice per group were sacrificed at 0.5, 1, 2, 4, 8 and 24 hours after the first dose of treatment (D1) and at the aforementioned time points with the addition of a 9 hour time point (i.e., 1 hour after the second daily dose) on Day 24 (D24) of treatment. Mice were anesthetized with chloroform and exsanguinated by cardiac puncture. Whole blood was collected on ice and then centrifuged to obtain serum. Acetonitrile was added to the samples before storage in gasketed screwcap tubes at -20°C. D24 samples were collected and processed in the same manner and stored at -20°C before being sent together with D1 samples to Pfizer (Groton, CT) for determination of drug concentrations. The supernatant was injected (10 µL injection volume) onto the LC (Shimadzu SCL-10A, Kyoto, Japan) - MS/MS (Sciex API 3000, Applied Biosystems Group, Foster City, CA) using a Hypersil C18 column (5µm, 50 x 2.1 mm, Thermo Electron Corp, Waltham, MA) and a step gradient consisting of a mobile phase of A: water with 0.05% 5mM ammonium formate and B: acetonitrile/water/5mM ammonium formate (80:20:0.05%). The ionization was electrospray positive ion mode. Bioanalytical data were captured using Analyst (Version 1.4.1, Applied Biosystems Group, Foster City, CA). Pharmacokinetic calculations were based on mean serum concentrations and performed using the non-compartmental approach (linear trapezoidal rule for AUC calculation with the aid of Watson 7.2 Bioanalytical LIMS [Thermo Electron Corp, Waltham, MA)). In the AUC calculations for D24, the plasma level of analytes at time zero was set to the 24-hour concentration. Concentrations of 0 were used for kinetic calculations for all results below the lower limit of quantitation (5 ng/mL).

Selected pharmacokinetic parameter values for the compound of formula (I) and LZD are presented in Table 2. The 130 mg/kg daily dose of LZD produced a steady state AUC of 379 µg-h/ml, approximately 50% higher than the anticipated steady state AUC observed in humans administered 600-625 mg by mouth twice daily, 215-294 µg-h/ml. (See Gee, T. et a; . Antimicrob Agents Chemother 45:1843-1846, 2001; Stalker DJ et al, J Antimicrob Chemother 51:1239-46, 2003.) Based on linear kinetics in this dose range, the 100 mg/kg dose of LZD in the mouse represents the upper end of the steady-state AUC range in humans, albeit with a Cₘₐₓ that is approximately 3 times higher than that obtained in humans. As expected there was significant first-pass metabolism of the compound of formula (I) to the major sulfoxide metabolite and the minor sulfone metabolite (Barbachyn et al. J Med Chem. 39:680-685, 1996). Because each of these metabolites has an MIC₉₀ of 0.5 µg/mL against *M*. tuberculosis, close to the MIC₉₀ of 0.25 µg/mL for the compound of formula (I), the concentration of each metabolite was added to that of the parent for the pharmacokinetic analyses. The steady-state AUC for the compound of formula (I) and its metabolites observed with the 100 mg/kg daily dose was approximately 3-fold lower than that observed with LZD at 130 mg/kg. The fact that the 25 mg/kg dose of the compound of formula (I) was as active as this dose of LZD suggests that the compound of formula (I) is 12 times more potent *in vivo* than LZD despite the similar MICs for both compounds.

**Table 2**

| Regimen | Cmax (µg/mL) | AUC₀₋₂₄ (µg-h/mL) |
|---|---|---|
| Compound of formula (I) 100 mg/kg | | |
| Day 1 | 6.07 | 7.33 |
| Day 24 | 4.32 | 8.74 |

| Sulfoxide metabolite | | |
|---|---|---|
| Day 1 | 20.0 | 37.8 |
| Day 24 | 16.9 | 98.8 |
| Sulfone metabolite | | |
| Day 1 | 0.79 | 2.58 |
| Day 24 | 0.66 | 9.73 |

| Compound of formula (I) + metabolites | | |
|---|---|---|
| Day 1 | 24.1 | 47.8 |
| Day 24 | 21.7 | 117 |

| LZD 130 mg/kg | | |
|---|---|---|
| Day 1 | 64.9 | 164 |
| Day 24 | 58.4 | 379 |

### Example 6

### In vivo activity of the combination

Mice were aerosol infected with 3.89 ± 0.19 log₁₀ CFU. Beginning 14 days after aerosol infection, when the mean lung CFU count was 7.37 ± 0.05 log₁₀, control mice received one of the following treatments: INH (25 mg/kg) alone, RIF (10 mg/kg) alone, RIF+PZA (150 mg/kg), RIF+INH+PZA, MXF (100 mg/kg)+PZA or RIF+MXF+PZA. Test mice received the compound of formula (I) (100 mg/kg) alone or added to each of the control regimens. Additional mice went untreated to serve as negative controls. Mice were sacrificed after 4 and 8 weeks of treatment for assessment of spleen weights and lung CFU counts. Untreated control mice died during the second month of the experiment.

The synergistic effect on lung CFU counts of adding the compound of formula (I) to a variety of 1-, 2- and 3-drug regimens is evident in Table 3.

**Table 3**

| | Lung log₁₀ CFU counts (±SD) | | | | |
|---|---|---|---|---|---|
| Regimen | Before treatment | After treatment with the indicated regimen | | After treatment with the indicated regimen plus the compound of formula (I) 100 mg/kg/day | |
| | Day 0 | Day 28 | Day 56 | Day 28 | Day 56 |
| No treatment | 7.37 ± 0.12 | 7.76 ± 0.09 | n.d. | 4.72 ± 0.17 | 2.70 ± 0.29 |
| INH | | 5.79 ± 0.14 | 4.53 ± 0.24 | 4.33 ± 0.29 | 2.87 ± 0.23 |
| RIF | | 5.74 ± 0.17 | 4.65 ± 0.24 | 3.73 ± 0.19 | 1.29 ± 0.22 |
| RIF-PZA | | 3.97 ± 0.11 | 1.05 ± 0.44 | 3.04 ± 0.22 | 0.50 ± 0.33 |
| RIF-INH-PZA | | 4.88 ± 0.09 | 2.47 ± 0.18 | 3.54 ± 0.24 | 0.47 ± 0.20 |
| RI F-MXF-PZA | | 3.72 ± 0.20 | 0.61 ± 0.38 | 3.41 ± 0.18 | 0.12 ± 0.27 |
| MXF-PZA | | 5.10 ± 0.13 | 3.17 ± 0.28 | 3.33 ± 0.33 | 0.93 ± 0.34 |

Monotherapy with the compound of formula (I) of the invention resulted in a 2.65 log₁₀ reduction in CFU counts from baseline to 4.72 log₁₀ over the first 28 days, whereas INH and RIF monotherapy reduced the lung CFU counts by 1.58 and 1.63 log₁₀ to 5.79 and 5.74 log₁₀, respectively. Remarkably, the compound of formula (I) of the invention continued to exert bactericidal activity during the second month of treatment, reducing the CFU count to 2.70 log₁₀ CFU greater than the activity of INH or RIF alone and similar to the CFU count in mice treated with the standard first-line combination regimen of RIF-INH-PZA which reduced the CFU count to 2.47 log₁₀. The combination of INH and the compound of formula (I) of the invention was no more active than the compound of formula (I) alone, while combining RIF with the compound of formula (I) had a multiplicative effect, resulting in a mean lung CFU count nearly 30 times lower than that observed with the compound of formula (I) alone. The combination of RIF-PZA had activity which reduced the mean lung CFU count to 1.05.

In this example, the addition of INH to RIF-PZA had a significant antagonistic effect, resulting in mean lung CFU counts of 1.05 ± 0.44 and 2.47 ± 0.18 after 2 months of treatment with RIF-PZA and RIF-INH-PZA, respectively. This effect could falsely suggest that substituting a new drug for INH in the RIF-INH-PZA regimen has a significant beneficial effect, even if the new drug is completely inactive itself, simply because the antagonistic effect of INH has been removed. In this example, however, the compound of formula (I) of the invention had similarly strong effects whether it was added to RIF-INH-PZA or substituted for INH (i.e., as RIF-PZA- compound of formula (I). After 2 months of treatment, the mean lung CFU counts were 0.47 ± 0.20 and 0.50 ± 0.33 in mice treated with RIF-INH-PZA-compound of formula (I) and RIF-PZA-compound of formula (I), respectively, resulting in a mean lung CFU count approximately 3.5 times lower than the mean lung CFU count in mice receiving RIF-PZA alone (p<0.05), providing further evidence that the benefit of substituting the compound of formula (I) of the invention for INH does not come from the removal of the antagonistic influence of INH. The compound of formula (I) of the invention also improved the activity of the RIF-MXF-PZA regimen. Treatment with RIF-MXF-PZA for 2 months resulted in a mean lung CFU count of 0.61 ± 0.38, with 1 of 5 mice culture-negative, while treatment with RIF-MXF-PZA- and the compound of formula (I) resulted in a mean CFU count of 0.12 ± 0.27, with 4 out of 5 mice culture-negative (complete eradication) (p= 0.05 for difference in mean CFU counts). The difference was of marginal statistical significance, likely due to the low CFU counts, but implies that the compound of formula (I) of the invention may further improve the regimen's sterilizing activity to prevent relapse. Finally, treatment with MXF-PZA-compound of formula (I) for 2 months resulted in a mean lung CFU count of 0.93 ± 0.34, resulting in a mean lung CFU count nearly 175 times lower than that observed without the compound of formula (I) demonstrating that the compound of formula (I) of the invention is able to replace RIF in the treatment-shortening RIF-MXF-PZA regimen without diminishing the regimen's activity.

### Example 7

### In vivo activity of the combination after prolonged administration and follow-up

Mice were aerosol infected with 4.45 ± 0.05 log₁₀ CFU. Beginning 14 days after aerosol infection, when the mean lung CFU count was 7.92 ± 0.15 log₁₀, control mice received RIF-INH-PZA for 8 weeks followed by RIF-INH for 8 weeks. One cohort of test mice received the same regimen to which the compound of formula (I) (160 mg/kg) was added for the entire 16 weeks duration, added for the first 8 weeks only, or added for the entire 16 weeks duration with the removal of INH after the first 8 weeks. Another cohort of test mice received the control regimen to which LZD was added for the entire 16 weeks duration or added for the first 8 weeks only. Additional mice went untreated to serve as negative controls. Mice were sacrificed after 8, 12 and 16 weeks of treatment for assessment of lung CFU counts. Additional mice were held without treatment for 12 weeks after completing 12 or 16 weeks of treatment. Untreated control mice died during the second month of the experiment.
The synergistic effect on lung CFU counts by adding the compound of formula (I) (Abbreviated as "U") to the control regimen during the first 8 weeks is evident in Table 4. The bactericidal effect of the control regimen was increased by 2 orders of magnitude. This effect is quantitatively and qualitatively different from the antagonistic effect of adding LZD.

**Table 4**

| Regimen | Lung log₁₀ CFU counts (± SD) after treatment for: | | Proportion (%) of mice with culture-positive relapse after treatment for: | |
|---|---|---|---|---|
| | 0 days | 8 wks | 12 wks | 16 wks |
| Untreated | 7.92 ± 0.15 | | | |
| 2 mo. RIF-INH-PZA + 2 mo. RIF-INH | | 3.17 ± 0.27 | | 18 of 20 (90%) |
| 2 mo. RIF-INH-PZA-U + 2 mo. RIF-INH-U | | 0.71 ± 0.26 | 9 of 20 (45%) | 1 of 20 (5%) |
| 2 mo. RIF-INH-PZA-U + 2 mo. RIF-U | | | 7 of 20 (35%) | 1 of 20 (5%) |
| 2 mo. RIF-INH-PZA-U + 2 mo. RIF-INH | | | 17 of 20 (85%) | 7 of 20 (35%) |
| 2 mo. RIF-INH-PZA-LZD + 2 mo. RIF-INH-LZD | | 4.28 ± 0.24 | | 20 of 20 (100%) |
| 2 mo. RIF-INH-PZA-LZD + 2 mo. RIF-INH | | | | 20 of 20 (100%) |

Treatment with regimen including the compound of formula (I) (abbreviated as "U" in Table 4) was associated with a lower likelihood of relapse (the gold standard measure of complete eradication) after completion of 12 and 16 weeks of treatment compared to mice receiving other regimens. After 12 weeks of treatment with the control regimen all mice remained culture-positive and would be expected to exhibit a 100% relapse rate. This is further supported by a 90% relapse rate after 16 weeks of treatment.

In contrast, groups receiving a regimen including the compound of formula (I) for the entire 16 weeks had an average relapse rate of 40% after 12 weeks and 5% after 16 weeks. When the compound formula (I) was added to the control regimen for only the first 8 weeks, 85% and 35% of mice relapsed after total treatment durations of 12 and 16 weeks, respectively. On the other hand, addition of LZD was antagonistic and prevented complete eradication in any mouse. Overall, these data demonstrate that the compound of formula (I) has synergistic effect when combined with the standard first-line regimen and is capable of shortening the duration of treatment by 1-to-2 months without sacrificing efficacy.

Hence, the compound of formula (I) has synergistic effect when combined with at least two agents useful for the treatment of tuberculosis. Most importantly, combining the compound of formula (I) with at least two agents for the treatment of tuberculosis dramatically increases bactericidal activity, suggesting that it may shorten the duration of chemotherapy for drug-susceptible TB as well as MDR-TB.

### Example 8

### Example 8A

### Four week dose ranging study in combination with standard first line agents, rifampin, isoniazid, and pyrazinamide

Example 8 describes the results of a study in which mice were infected with *Mycobacterium tuberculosis* similar to the manner described in Example 6 above. Other than the controls, all of the mice were treated with the following three drugs:
1) rifampin, 10mg/kg(R),
2) isoniazid, 25mg/kg(H), and
3) pyrazinamide,150mg/kg(Z).

The mice were divided into eight groups (8) and six of the groups received varying doses of the compound of Formula I("U"). Doses ranged from 12.5mg/kg to 160mg/kg and are listed in Table 5 below.

The study was carried out in the following manner. Six-week-old female BALB/c mice were infected by the aerosol route with *Mycobacterium tuberculosis* H37Rv. On that day (D-17), 5 mice were sacrificed to determine the number of CFU implanted in the lungs. Seventeen days later (D0), 5 additional mice were sacrificed to determine the baseline CFU count in the lungs. The remaining mice were randomized to treatment groups, as indicated in Table 5, and started on the specified drug regimen. Treatment was administered once daily, 5 days per week, except for group 8, which received thrice weekly treatment (3/7) throughout. At the conclusion of the 4 week test period, all of the animals were sacrificed for lung CFU counts, except for 80 animals from the Group 4, below. These animals were utilized in an 8 week protocol which is described below in Example 8B.

**Table 5**

| **Regimen** | # Mice | CFU Count Day (-) 17 | CFU Count Day 0 | CFU Count 4 weeks |
|---|---|---|---|---|
| **Controls** | | | | |
| 1) Untreated | 15 | 3.50 ± 0.23 | 7.71 ± 0.06 | dead |
| 2) RHZ | 5 | | | 4.71 ± 0.17 |

| **Test Regimen** | | | | |
|---|---|---|---|---|
| 3)RHZU _{12.5} | 5 | | | 4.51 ± 0.16 |
| 4) RHZU₂₅ | 85 | | | 4.19 ± 0.26 |
| 5) RHZU₅₀ | 5 | | | 4.32 ± 0.26 |
| 6) RHZU₁₀₀ | 5 | | | 4.26 ± 0.25 |
| 7) RHZU₁₆₀ | 5 | | | 4.01 ± 0.21 |
| 6) RHZU₅₀(3/7) | 5 | | | 4.88 ± 0.33 |

As depicted above, the compound formula I demonstrated a dose dependent effect on CFU numbers. The addition of this compound to the standard regimen of RHZ produced further reduction in the number of TB colonies contained within the lung.

### Example 8B

### Eight week dose ranging study

Eighty of the mice from Group 4 (in Example 8A) that had been receiving 25mg/kg of the compound of formula I(U) along with rifampin(R), isoniazid(H), and pyrazinamide(Z) were evaluated in the second phase of the study. They were assigned to one of the groups described below in Table 6 and the study was continued for an additional 4 weeks (i.e. 8 week results).

**Table 6**

| **Drug Regimen** | **# of Mice** |
|---|---|
| 1) No treatment | 5 |
| 2) RH | 5 |
| 3) R | 5 |
| 4) H | 5 |

| **U given with or without R:** | |
|---|---|
| 5) U₁₆₀+/-R | 5 |
| 6) U₁₀₀+/-R | 5 |
| 7) U₅₀+/-R | 5 |
| 8) U₂₅+/-R | 5 |
| 9) U_{12.5}+/-R | 5 |
| 10) U₅₀(3/7) +/-R | 5 |

As described above, 5 animals served as the control and no further drugs were given. Five were given the combination of rifampin(R) and isoniazid (H), five given isoniazid (H) or rifampin(R) alone. Of the twelve (12) remaining groups, all were given with the compound of formula (I) in doses ranging from 12.5 mg/kg to 160 mg/kg. Six of these groups were also dosed with rifampin (R). The rifampin and isoniazid was dosed in the same manner and in the same amount as in Example 8A.

At the conclusion of the eight (8) week experiment, all of the animals were sacrificed and CFU lung counts were obtained. The following results were obtained:

**Table 7**

| **Treatment** | **Lung CFU** |
|---|---|
| 1) Untreated | 5.49 ± 0.23 |
| 2) Isoniazid(H) | 4.46 ± 0.37 |
| 3) Rifampin(R) | 4.18 ± 0.55 |
| 4) R and H | 3.84 ± 0.46 |

At the initiation of this second phase, the eighty animals had a mean CFU of 4.19 + 0.26 (based upon the other mice from Group 4 that were sacrificed). Table 7 shows the results obtained with control group, the rifampin(R) only, isoniazid(H) only and the combinatinn of both isoniazid(H) and rifampin(R).
Table 8 shows the results obtained with the mice receiving the compound of formula (I)(U) alone or in combination with rifampin(R).

**Table 8**

| **Dose of U** | **CFU-U only** | **CFU- U and R** |
|---|---|---|
| U₁₂₋₅ | 4.99 ± 0.19 | 3.42 ± 0.40 |
| U₂₅ | 4.26 ± 0.24 | 2.83 ± 0.24 |
| U₅₀ | 3.56 ± 0.15 | 2.49 ± 0.31 |
| U₁₀₀ | 2.88 ± 0.34 | 1.54 ± 0.45 |
| U₁₆₀ | 2.42 ± 0.31 | 1.26 ± 0.53 |
| U₅₀(3/7) | 4.21 ± 0.26 | |

The compound of Formula I(U) demonstrated dose-dependent bactericidal activity against persisting tubercle bacilli that remained viable after the initial 4 weeks of treatment with RHZU₂₅. The following effects were observed with U monotherapy: a growth-inhibitory effect at 12.5 mg/kg, a bacteriostatic effect at 25 mg/kg, a >0.5 log reduction at 50 mg/kg, a >1 log reduction at 100 mg/kg, and a nearly 2 log reduction at 160 mg/kg. The combination of rifampin(R) and the compound of formula (I) U was synergistic and proved to have strong bactericidal activity against persisters. When the compound of formula (I) U was administered at doses ≥25 mg/kg, the combination of it and rifampin R was more effective than the standard regimen of rifampin(R) and isoniazid (H). Results with U alone support the conclusion that U-containing regimens may be capable of significantly shortening the duration of treatment for both multidrug-resistant tuberculosis and extensively drug-resistant tuberculosis.

### Example 8C

### Additional Dose Ranging Study

The protocol described above in Example 8A and 8B was carried out on an alternative occasion. The only substantive changes were the number of animals per group and the initial incubation period. Table 9 provides the results obtained

**Table 9**

| **Regimen** | **# Mice** | **CFU Count Day (-) 12** | **CFU Count Day 0** | **CFU Count 4 weeks** |
|---|---|---|---|---|
| **Controls** | | | | |
| 1) Untreated | 12 | 3.26 ± 0.14 | 6.28 ± 0.08 | 7.23 ± 0.15 |
| 2) RHZ | 4 | | | 4.58 ± 0.15 |

| **Test Regimen** | | | | |
|---|---|---|---|---|
| 1)RHZU₁₆₀ | 4 | | | 4.16 ± 0.28 |
| 2) RHZU₁₀₀ | 64 | | | 3.93 ± 0.21 |
| 3) RHZU₅₀ | 4 | | | 4.30 ± 0.27 |
| 4) RHZU₂₅ | 4 | | | 4.39 ± 0.23 |
| 5) RHZU_{12.5} | 4 | | | 4.30 ± 0.25 |
| 6) RHZU_{6.25} | 4 | | | 4.39 |

Testing continued for an additional 4 weeks with 60 of the mice from Test Group 2 immediately above. The mice either received U alone, at varying doses, or the combination of U and R. The following results were obtained.

**Table 10**

| **Regimen** | # Mice | CFU Count 8 weeks |
|---|---|---|
| **Controls** | | |
| 1) Untreated | 4 | 4.91 ± 0.17 |
| 2) R | 4 | 4.16 ± 0.65 |
| 3) RH | 4 | 3.00 ± 0.16 |

| **Test Regimen** | | |
|---|---|---|
| 1) U₁₆₀ | 4 | 2.90 ± 0.32 |
| 2) RU₁₆₀ | 64 | 2.16 ± 0.20 |
| 3) U₁₀₀ | 4 | 3.86 ± 0.53 |
| 4) RU₁₀₀ | 4 | 2.35 ± 0.21 |
| 5) U₅₀ | 4 | 4.59 ± 0.28 |
| 6) RU₅₀ | 4 | 3.17 ± 0.76 |
| 7) U₂₅ | 4 | 5.27 ± 0.20 |
| 8) RU₂₅ | 4 | 4.27 ± 0.33 |
| 9) U_{12.5} | 4 | 5.30 ± 0.08 |
| 10) RU_{12.5} | 4 | 3.62 ± 0.40 |
| 11) U_{6.25} | 4 | 4.93 ± 0.32 |
| 12) RU_{6.25} | 4 | 4.38 ± 0.16 |

As depicted above, the compound formula I demonstrated a dose dependent effect on CFU numbers. The addition of this compound to the standard regimen of rifampin(R) and isoniazid (H) and rifampin (R) alone produced further reduction in the number of TB colonies contained within the lung at both 4 and 8 weeks.

### Example 9

In Example 9 the impact of the compound of formula I on existing first and second line anti-tuberculosis drugs was evaluated. Two doses of the compound were evaluated, 25 mg/kg and 100 mg/kg. The following standard anti-tuberculosis agents were evaluated, at the specified doses:
1) isoniazid, 10mg/kg (H)
2) rifampin, 10mg/kg (R)
3) pyrazinamide, 150mg/kg, (Z)
4) ethambutol 100mg/kg (Eb)
5) moxifloxacin 100mg (M)
6) ethionamide 50mg/kg (Et)
7) amikacin 150mg/kg subcutaneously, (A)
8) cycloserine 250mg/kg twice daily (Cs)
9) para-aminosalicyclic acid 750mg/kg (Ps)
10) capreomycin 125mg/kg subcutaneously (Cap)
11) clofazimine 20mg/kg (C)

The study was carried out in the following manner:

### Methods

Mice: female BALB/c, 6 weeks old
Infection: aerosol infection with ~10⁴ CFU *M. tuberculosis* H37Rv followed by randomization to treatment group
Treatment: initiated on day 14 after infection (D0) when the CFU counts in the mouse lungs was ~10⁸ CFU.
All drugs were given once daily (except cycloserine - twice daily) by the oral route (except amikacin and capreomycin - subcutaneous injection), 5 days per week.
Mouse sacrifices for CFU counts in lungs: According to the experimental scheme, 4 mice were sacrificed the day after infection (D-13) and 2 weeks later (D0) to assess the number of implanted CFU and the baseline CFU counts on treatment initiation, respectively. The activity of each regimen was be assessed by lung CFU counts after 4 weeks of treatment.

### Results

Mice were infected with - 4.5 log₁₀ CFU. Fourteen days later the lung CFU count at the initiation of treatment on day zero was approximately 8.3 log₁₀. Untreated mice died within the first 3 weeks of infection (Table 11). The survival of mice treated with pyrazinamide (Z), cycloserine (Cs), and capreomycin (Cap) was not significantly different from untreated controls. Treatment with para-aminosalicylic acid (Ps) delayed but did not prevent mortality. Treatment with ethambutol (Eb) and clofazimine (C) prevented some, but not all, mortality. Treatment with isoniazid (H), rifampin (R), moxifloxacin (M), amikacin (A) and ethionamide (Et) prevented death, as did treatment with the compound of formula I (U) at either dose, whether alone or in combination with other drugs.

**Table 11**

| Treatment group | Proportion (%) surviving to Day 42 post-infection | Time to 50% mortality (days) |
|---|---|---|
| Any U-containing regimen | 4/4 (100%) | N/A |
| H, R, M, A or Et | 4/4 (100%) | N/A |
| C | 3/4 (75%) | N/A |
| Eb | 2/4 (50%) | 30 |
| Ps | 0/4 (0%) | 31 |
| Cap | 0/4 (0%) | 22 |
| Cs | 0/4 (0%) | 22 |
| Z | 0/4 (0%) | 21 |
| No treatment | 0/4 (0%) | 21 |

In addition to measuring survival, lung CFU counts were also determined as described above and are reported below in Table 12

**Table 12**

| | Mean lung log₁₀ CFU count (±) S.D. | | | |
|---|---|---|---|---|
| Regimen | at treatment initiation (D0) | after 4 weeks of treatment with: | | |
| | | Regimen alone | Regimen + U 25 mg/kg | Regimen + U 100 mg/kg |
| 1) Untreated | 8.36 ± 0.10 | N/A | 7.17 ± 0.23 | 5.40 ± 0.10 |
| 2) H | | 7.01 ± 0.14 | 6.22 ± 0.06 | 5.79 ± 0.33 |
| 3) R | | 6.23 ± 0.11 | 5.91 ± 0.21 | 5.02 ± 0.10 |
| 4) Z | | N/A | 4.73 ± 0.38 | ≤ 3.59*** |
| 5) Eb | | 7.41 ± 0.57* | 6.86 ± 0.16 | 5.50 ± 0.13 |
| 6) M | | 6.49 ± 0.12 | 7.08 ± 0.10 | 5.33 ± 0.30 |
| 7) Et | | 7.19 ± 0.04 | 7.18 ± 0.18 | 5.46 ± 0.15 |
| 8) A | | 7.24 ± 0.14 | 6.78 ± 0.16 | 5.18 ± 0.17 |
| 9) Cs | | N/A | 7.19 ± 0.21 | 5.66 ± 0.11 |
| 10) Ps | | N/A | 6.87 ± 0.21** | 5.39 ± 0.32 |
| 11) Cap | | N/A | 6.81 ± 0.13 | 5.35 ± 0.17 |
| 12) C | | 5.92 ± 0.24** | 6.25 ± 0.20 | 4.91 ± 0.14 |

| | | | | |
|---|---|---|---|---|
| N/A, not available due to death in 4 of 4 mice before W4; * 2 of 4 mice survived; ** 3 of 4 mice survived; *** the CFU counts was below the lower limit of detection of 3.40 for 2 of 4 mice | | | | |

A review of the data shows that the addition of the compound of Formula I (U), significantly reduced bacterial load in a dose dependent manner when added to standard anti-tubercular drugs. These results suggest that the compound of formula I could be used to improve the efficacy of treatment regimens for drug resistant tuberculosis (either multidrug-resistant or extensively drug-resistant).

## Claims

1. Use of a compound of the formula or a pharmaceutically acceptable salt thereof: 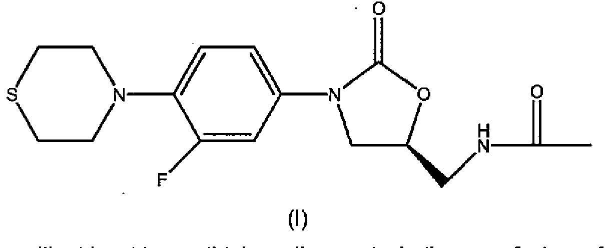 in combination with at least two anti-tuberculin agents in the manufacture of a medicament for the treatment of tuberculosis.

2. The use of claim 1, wherein said at least two agents is selected from the group consisting of isoniazid, rifampin, rifapentine, rifabutin, pyrazinamide, ethambutol, streptomycin, kanamycin, amikacin, moxifloxacin, gatifloxacin, levofloxacin, ofloxacin, ciprofloxacin, capreomycin, ethionamide, cycloserine, para-aminosalicylic acid, thiacetazone, clarithromycin, amoxicillin-clavulanic acid, imipenem, meropenem, viomycin, terizidone, TMC207, PA-824, OPC-7683, LL-3858 and SQ-109.

3. The use of claim 1, wherein one of said at least two agents is selected from the group consisting of isoniazid, rifampin, rifapentine, rifabutin, pyrazinamide, moxifloxacin, gatifloxacin, levofloxacin, ofloxacin, ciprofloxacin, and ethambutol.

4. The use of claim 1, wherein one of said at least two agents are selected from the group consisting of pyrazinamide, rifampin, rifapentine and isoniazid.

5. Use of a of a compound of formula (I) or a pharmaceutically acceptable salt thereof: 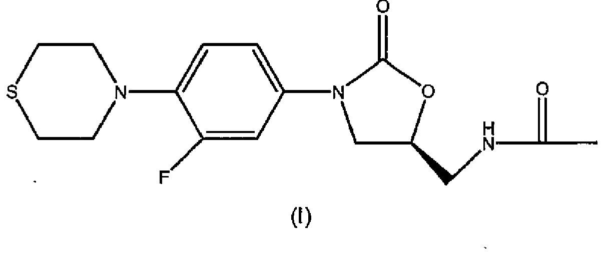 in combination with at least one anti-tuberculin agent in the manufacture of a medicament for treating tuberculosis after a subject has undergone an initial phase of treatment, for tuberculosis.

6. The use of claim 5, wherein said at least one agent is selected from the group consisting of rifampin, rifapentine, rifabutin, pyrazinamide, ethambutol, streptomycin, kanamycin, amikacin, moxifloxacin, gatifloxacin, levofloxacin, ofloxacin, ciprofloxacin, capreomycin, ethionamide, cycloserine, para-aminosalicylic acid, thiacetazone, clarithromycin, amoxicillin-clavulanic acid, imipenem, meropenem, viomycin, terizidone, TMC207, PA-824, OPC-7683, LL-3858 and SQ-109.

7. The use of claim 5 or 6 wherein said active tuberculosis is selected from the group consisting of drug-sensitive tuberculosis, mono-drug resistant tuberculosis, multi-drug-resistant tuberculosis (MDR) and extensively drug-resistant tuberculosis (XDR).

8. A pharmaceutical composition comprising:
i) a therapeutically effective amount of a compound of formula (I) or a pharmaceutically acceptable salt thereof: 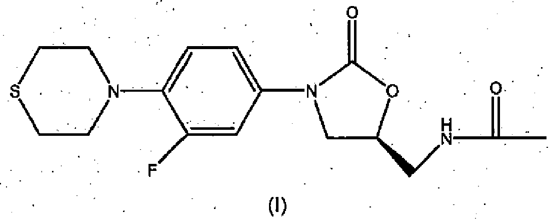
(ii) a therapeutically effective amount of at least two agents useful in the treatment of tuberculosis and,
(iii) one or more pharmaceutically acceptable carriers or vehicles.

9. The pharmaceutical composition of claim 8, wherein said at least one agent is selected from the group consisting of isoniazid, rifampin, rifapentine, rifabutin, pyrazinamide, ethambutol, streptomycin, kanamycin, amikacin, moxifloxacin, gatifloxacin, levofloxacin, ofloxacin, ciprofloxacin, capreomycin, ethionamide, cycloserine, para-aminosalicylic acid, thiacetazone, clarithromycin, amoxiciflin-clavulanic acid, imipenem, meropenem, viomycin, terizidone, TMC207, PA-824, OPC-7683, LL-3858 and SQ-109.

10. The pharmaceutical composition of claim 8 or 9, wherein said at least one agent is selected from the group consisting of isoniazid, rifampin, rifapentine, rifabutin, pyrazinamide, moxifloxacin, gatifloxacin, levofloxacin, ofloxacin, and ethambutol.

## Patentansprüche

1. Verwendung einer Verbindung der folgenden Formel oder eines pharmazeutisch zulässigen Salzes davon: 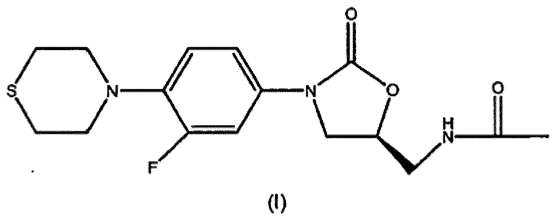 in Kombination mit mindestens zwei Anti-Tuberkulinmitteln bei der Herstellung eines Medikaments zur Behandlung von Tuberkulose.

2. Verwendung nach Anspruch 1, wobei die mindestens zwei Mittel aus der Gruppe ausgewählt sind, die aus Isoniazid, Rifampin, Rifapentin, Rifabutin, Pyrazinamid, Ethambutol, Streptomycin, Kanamycin, Amikacin, Moxifloxacin, Gatifloxacin, Levofloxacin, Ofloxacin, Ciprofloxacin, Capreomycin, Ethionamid, Cycloserin, Paraaminosalicylsäure, Thiacetazon, Clarithromycin, Amoxicillin-Clavulansäure, Imipenem, Meropenem, Viomycin,Terizidon, TMC207, PA-824, OPC-7683, LL-3858 und SQ-109 besteht.

3. Verwendung nach Anspruch 1, wobei eines der mindestens zwei Mittel aus der Gruppe ausgewählt ist, die aus Isoniazid, Rifampin, Rifapentin, Rifabutin, Pyrazinamid, Moxifloxacin, Gatifloxacin, Levofloxacin, Ofloxacin, Ciprofloxacin und Ethambutol besteht.

4. Verwendung nach Anspruch 1, wobei eines der mindestens zwei Mittel aus der Gruppe ausgewählt ist, die aus Pyrazinamid, Rifampin, Rifapentin und Isoniazid besteht.

5. Verwendung einer Verbindung der Formel (I) oder eines pharmazeutisch zulässigen Salzes davon: 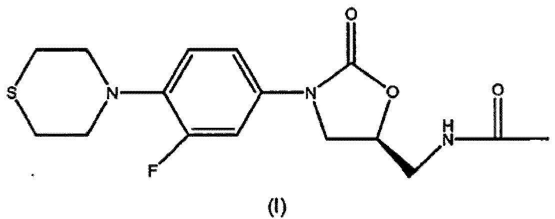 in Kombination mit mindestens einem Anti-Tuberkulinmittel bei der Herstellung eines Medikaments zur Behandlung von Tuberkulose, nachdem ein Individuum eine erste Phase einer Tuberkulosebehandlung durchlaufen hat.

6. Verwendung nach Anspruch 5, wobei das mindestens eine Mittel aus der Gruppe ausgewählt ist, die aus Rifampin, Rifapentin, Rifabutin, Pyrazinamid, Ethambutol, Streptomycin, Kanamycin, Amikacin, Moxifloxacin, Gatifloxacin, Levofloxacin, Ofloxacin, Ciprofloxacin, Capreomycin, Ethionamid, Cycloserin, Paraaminosalicylsäure, Thiacetazon, Clarithromycin, Amoxicillin-Clavulansäure, Imipenem, Meropenem, Viomycin,Terizidon, TMC207, PA-824, OPC-7683, LL-3858 und SQ-109 besteht.

7. Verwendung nach Anspruch 5 oder 6, wobei die aktive Tuberkulose aus der Gruppe ausgewählt ist, die aus arzneimittelempfindlicher Tuberkulose, Monodrug-resistenter Tuberkulose, Multidrug-resistenter Tuberkulose (MDR) und stark arzneimittelresistenter Tuberkulose (XDR) besteht.

8. Pharmazeutische Zusammensetzung, umfassend:
(i) eine therapeutisch wirksame Menge einer Verbindung der Formel (I) oder eines pharmazeutisch zulässigen Salzes davon; 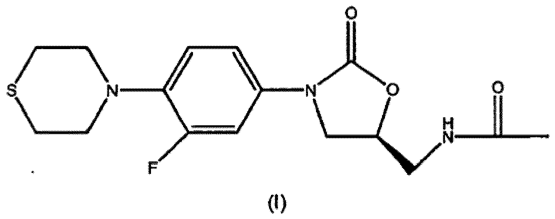
(ii) eine therapeutisch wirksame Menge von mindestens zwei Mitteln, die bei der Behandlung von Tuberkulose nützlich sind, und
(iii) mindestens einen pharmazeutisch zulässigen Träger oder mindestens ein pharmazeutisch zulässiges Vehikel.

9. Pharmazeutische Zusammensetzung nach Anspruch 8, wobei das mindestens eine Mittel aus der Gruppe ausgewählt ist, die aus Isoniazid, Rifampin, Rifapentin, Rifabutin, Pyrazinamid, Ethambutol, Streptomycin, Kanamycin, Amikacin, Moxifloxacin, Gatifloxacin, Levofloxacin, Ofloxacin, Ciprofloxacin, Capreomycin, Ethionamid, Cycloserin, Paraaminosalicylsäure, Thiacetazon, Clarithromycin, Amoxicillin-Clavulansäure, Imipenem, Meropenem, Viomycin, Terizidon, TMC207, PA-824, OPC-7683, LL-3858 und SQ-109 besteht.

10. Pharmazeutische Zusammensetzung nach Anspruch 8 oder 9, wobei das mindestens eine Mittel aus der Gruppe ausgewählt ist, die aus Isoniazid, Rifampin, Rifapentin, Rifabutin, Pyrazinamid, Moxifloxacin, Gatifloxacin, Levofloxacin, Ofloxacin und Ethambutol besteht.

## Revendications

1. Utilisation d'un composé de formule (I), ou d'un de ses sels pharmaceutiquement acceptables : 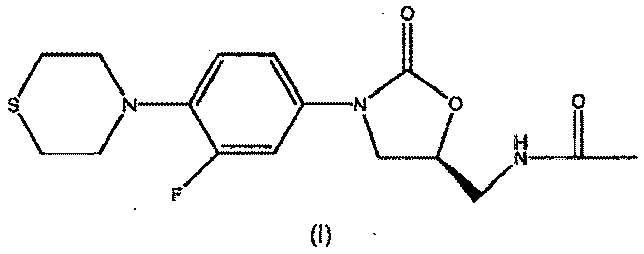 en combinaison avec au moins deux agents antituberculeux dans la fabrication d'un médicament destiné au traitement de la tuberculose.

2. Utilisation selon la revendication 1, dans laquelle lesdits au moins deux agents sont choisis dans le groupe constitué par l'isoniazide, la rifampine, la rifapentine, la rifabutine, le pyrazinamide, l'éthambutol, la streptomycine, la kanamycine, l'amikacine, la moxifloxacine, la gatifloxacine, la lévofloxacine, l'ofloxacine, la ciprofloxacine, la capréomycine, l'éthionamide, la cyclosérine, l'acide para-aminosalicylique, la thiacétazone, la clarithromycine, l'amoxicilline-acide clavulanique, l'imipénème, le méropénème, la viomycine, la térizidone, le TMC207, le PA-824, l'OPC-7683, le LL3858 et le SQ-109.

3. Utilisation selon la revendication 1 dans laquelle l'un desdits au moins deux agents est choisi dans le groupe constitué par l'isoniazide, la rifampine, la rifapentine, la rifabutine, le pyrazinamide, la moxifloxacine, la gatifloxacine, la lévofloxacine, l'ofloxacine, la ciprofloxacine et l'éthambutol.

4. Utilisation selon la revendication 1 dans laquelle l'un desdits au moins deux agents est choisi dans le groupe constitué par le pyrazinamide, la rifampine, la rifapentine et l'isoniazide.

5. Utilisation d'un composé de formule (I) ou d'un de ses sels pharmaceutiquement acceptables : 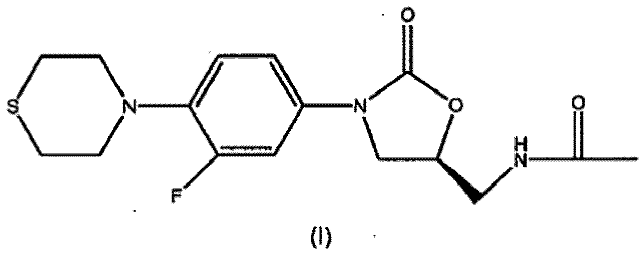 en combinaison avec au moins un agent antituberculeux, dans la fabrication d'un médicament destiné à traiter la tuberculose lorsqu'un sujet a été soumis à une phase initiale de traitement pour la tuberculose.

6. Utilisation selon la revendication 5 dans laquelle ledit au moins un agent est choisi dans le groupe constitué par la rifampine, la rifapentine, la rifabutine, le pyrazinamide, l'éthambutol, la streptomycine, la kanamycine, l'amikacine, la moxifloxacine, la gatifloxacine, la lévofloxacine, l'ofloxatine, la ciprofloxacine, la capréomycine, l'éthinamide, la cyclosérine, l'acide para aminosalicylique, la thiacétazone, la clarithromycine, l'amoxicilline-acide clavulanique, l'imipénème, le méropénème, la viomycine, la térizidone, le TMC207, le PA-824, l'OPC-7683, le LL-3858 et le SQ-109.

7. Utilisation selon les revendications 5 ou 6 dans laquelle ladite tuberculose active est sélectionnée dans le groupe constitué par la tuberculose réactive aux médicaments, la tuberculose mono-résistante aux médicaments, la tuberculose multi-résistante aux médicaments (MDR) et la tuberculose ultra-résistante aux médicaments (XDR).

8. Composition pharmaceutique comprenant :
i) une quantité efficace d'un point de vue thérapeutique d'un composé de formule (I), ou d'un de ses sels pharmaceutiquement acceptable : 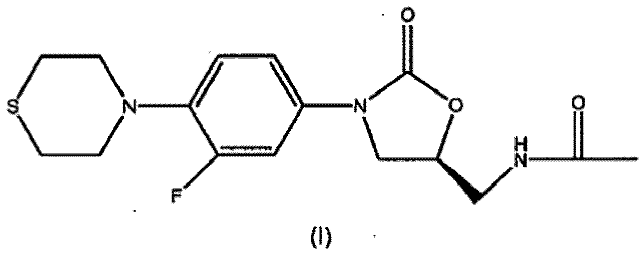
ii) une quantité efficace d'un point de vue thérapeutique d'au moins deux agents utiles dans le traitement de la tuberculose, et
iii) un ou plusieurs supports ou véhicules pharmaceutiquement acceptables.

9. Composition pharmaceutique selon la revendication 8 dans laquelle ledit au moins un agent choisi dans le groupe constitué par l'isoniazide, la rifampine, la rifapentine, la rifabutine, le pyrazinamide, l'éthambutiol, la streptomycine, la kanamycine, l'amikacine, la moxifloxacine, la gatifloxacine, la lévofloxacine, l'ofloxacine, la ciprofloxacine, la capréomycine, l'éthionamide, la cyclosérine, l'acide para aminosalicylique, la thiacétazone, la clarithromycine, l'acide amoxicilline-clavulanique, l'imipénème, le méropénème, la viomycine, la térizidone, le TMC207, le PA-824, l'OPC-7683, le LL-3858 et le SQ-109.

10. Composition pharmaceutique selon les revendications 8 ou 9 dans laquelle ledit au moins un agent est choisi dans le groupe constitué l'isoniazide, la rifampine, la rifapentine, la rifabutine, le pyrazinamide, la moxifloxacine, la gatifloxacine, la lévofloxacine, l'ofloxacine et l'éthambutol.
